# NEUE EUROPÄISCHE PATENTSCHRIFT

(11) **EP 2 266 465 B2**
(45) Veröffentlichungstag und Bekanntmachung des Hinweises auf die Entscheidung über den Einspruch: **26.07.2023**
(45) Hinweis auf die Patenterteilung: 21.05.2014
(21) Anmeldenummer: 09169783.9
(22) Anmeldetag: 22.03.2007
(51) Int. Cl.: A61B 17/00, A61B 17/12

(54) **Occlusionsinstrument und Verfahren zu dessen Herstellung**
Occlusion instrument and method for its production
Dispositif d'occlusion et son procédé de fabrication

(30) Priorität: 24.03.2006 DE 102006013770
(43) Veröffentlichungstag der Anmeldung: 29.12.2010
(62) Teilanmeldung aus: 07723515.8
(73) Patentinhaber: Occlutech Holding AG, 8200 Schaffhausen (CH)
(72) Erfinder: Figulla, Hans-Reiner, Prof., 07749 Jena (DE); Moszner, Robert, Dr., 07639 Bad Klosterlausnitz (DE); Krizanic, Florian, Dr., 07743 Jena (DE); Moszner, Friedrich, 99441 Hohlstedt (DE)
(74) Vertreter: KIPA AB

(56) Entgegenhaltungen:
- EP-B1- 0 957 773
- WO-A-99/12478
- WO-A-2004/064671
- WO-A-2005/020822
- WO-A1-97/42878
- WO-A1-98/47430
- WO-A1-99/39646
- CN-A- 1 736 346
- CN-Y- 2 524 710
- CN-Y- 2 613 248
- DE-B3- 10 338 702
- US-A- 5 725 552

## Beschreibung

Die vorliegende Erfindung betrifft ein Occlusionsinstrument, welches aus einer Fassung und einem Geflecht dünner Drähte oder Fäden besteht, das mittels eines Umformungs- und Wärmebehandlungsverfahrens eine geeignete Form erhält, wobei das Occlusionsinstrument einen proximalen Retentionsbereich, einen distalen Retentionsbereich, wobei in dem distalen Retentionsbereich die Enden der Drähte in einer Fassung zusammenlaufen, und einen zylindrischen Steg zwischen dem proximalen und dem distalen Retentionsbereich aufweist, wobei die beiden Retentionsbereiche durch einen meist intravaskulären Operationseingriff beiderseits eines zu verschließenden Shunts in einem Septum zur Anlage kommen, während der Steg durch den Shunt hindurch läuft. Die Erfindung betrifft des weiteren ein Verfahren zur Herstellung dieses Occlusionsinstruments.

In der Medizintechnik besteht seit längerem das Bemühen, septale Defekte, wie etwa Defekte des Vorhofseptums, mittels eines transvenösen, interventionellen Zugangs nichtchirurgisch, also ohne Operation im eigentlichen Sinne, katheterinterventionell zu verschließen. Dabei wurden verschiedene Occlusionssysteme mit unterschiedlichen Vor- und Nachteilen vorgeschlagen, ohne das sich bisher ein bestimmtes Verschluss-System durchsetzen konnte. Im folgenden werden die verschiedenen Systeme "Occluder" oder "Occlusionsinstrumente" genannt.

Bei allen interventionellen Occlusionssystemen wird transvenös über einen in einem Septum vorliegenden, zu verschließenden Defekt ein selbst-expandierendes Schirmsystem eingebracht. Ein derartiges System könnte beispielsweise aus zwei Schirmchen bestehen, die jeweils an der distalen Seite (d.h. an der weiter von der Körpermitte bzw. vom Herzen entfernten Seite) bzw. an der proximalen Seite (d.h. an der näher zur Körpermitte angeordneten Seite) des Septums positioniert werden, wobei anschließend die beiden Schirmprothesen im Septum-Defekt zu einem Doppelschirm verschraubt werden. Das Verschluss-System besteht somit dann im zusammengebauten Zustand üblicherweise aus zwei aufgespannten Schirmchen, die über einen kurzen, durch den Defekt hindurch laufenden Stift miteinander verbunden sind.

Bei derartigen aus dem Stand der Technik bekannten Occlusionsinstrumenten stellt es sich jedoch als nachteilig heraus, dass die Implantationsprozedur relativ kompliziert, schwierig und aufwendig ist. Abgesehen von dem komplizierten Implantieren des Verschluss-Systems im zu verschließenden Septum-Defekt besteht bei den verwendeten Schirmchen grundsätzlich die Gefahr der Materialermüdung mit Branchenfraktur. Ferner ist häufig mit thrombembolischen Komplikationen zu rechnen.

Bei einem anderen Typ eines Occlusionsinstruments, dem sogenannten Lock-Clamshell-Schirmsystem, sind zwei vorzugsweise mit Dacron bespannte Stahlschirme vorgesehen, die durch je vier Ärmchen stabilisiert werden. Dieser Occludertyp wird über einen venösen Zugang des Patienten implantiert. Bei dem Lock-Clamshell-Occluder hat es sich jedoch als problematisch erwiesen, dass das zur Implantation benötigte Einführbesteck relativ groß ausgeführt werden muss. Ein weiterer Nachteil liegt darin, dass viele verschiedene Occludergrößen benötigt werden, um den jeweiligen Proportionen des zu schließenden Septum-Defekts gerecht zu werden. So hat sich herausgestellt, dass die Schirmchen im eingesetzten Zustand nicht vollständig abflachen, wenn die Länge oder der Durchmesser des im Defekt eingesetzten Stegs nicht optimal passt. Dies führt zu einer unvollständigen Endothelialisierung. Ferner hat es sich gezeigt, dass viele der im Körper des Patienten implantierten Systeme über einen längeren Zeitraum aufgrund der erheblichen mechanischen Belastung Materialermüdungen und Brüche in den metallischen Strukturen aufweisen. Dies ist insbesondere dann der Fall, wenn zwischen dem Implantat und dem Septum dauerhaft Spannungen bestehen.

Um diese Nachteile auszuräumen, wurden selbst-zentrierende Occlusionsinstrumente entwickelt, die mittels minimalinvasiver Verfahren, beispielsweise über einen Katheder und Führungsdrähte, in den Körper des Patienten eingeführt und in den zu verschließenden Septum-Defekt eingebracht werden. Der Konstruktion liegt dabei das Prinzip zugrunde, dass sich das Occlusionsinstrument auf die Größe des für den intravaskulären Operationseingriff verwendeten Einführbesteckes bzw. Katheder verjüngen lässt. Ein derart verjüngtes Occlusionsinstrument wird dann über den Katheder in den zu verschließenden Septum-Defekt bzw. in den zu verschließenden Shunt des Septum-Defektes eingebracht. Danach tritt der Occluder aus dem Katheder aus, worauf sich anschließend die selbst-expandierenden Schirmchen bzw. Retentionsscheibchen entfalten, die sich beiderseits des Septums anlegen. Die Schirme wiederum enthalten beispielsweise aus Dacron gefertigte Gewebeeinlagen oder werden von solchen überspannt, womit der Defekt bzw. Shunt verschlossen wird. Die im Körper verbleibenden Implantate werden nach einigen Wochen bis Monaten mehr oder weniger vollständig von körpereigenem Gewebe eingeschlossen.

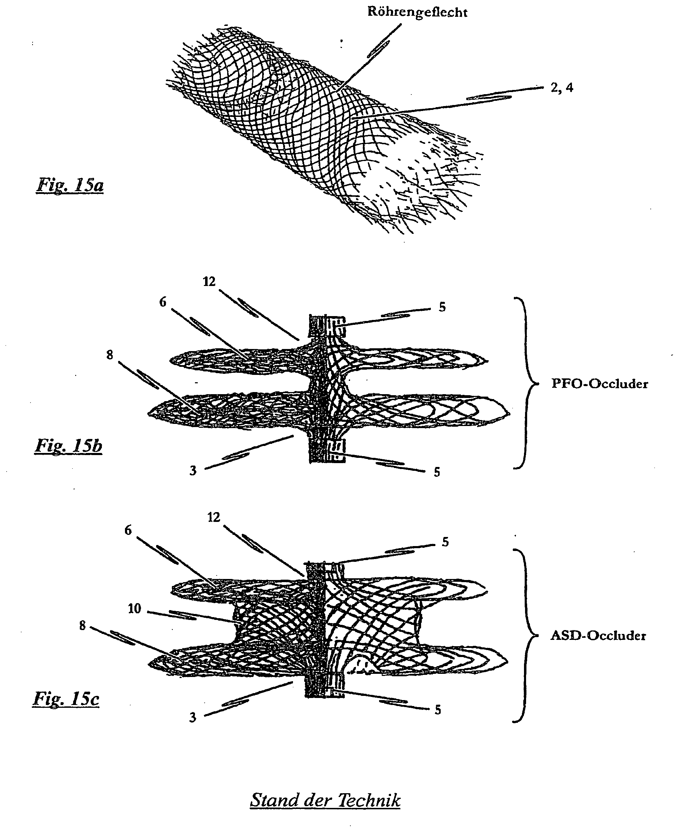

Ein Beispiel eines derartigen selbst-zentrierenden Occlusionsinstruments ist aus der US-Patentschrift Nr. 5,725,552 bekannt, in der ein unter dem Namen "Amplatzer-Occluder" bekanntes Occlusionsinstruments beschrieben ist. Dieses bekannte System soll nachfolgend kurz unter Bezugnahme auf die Fig. 15a bis Fig. 15c beschrieben werden. Im einzelnen ist Fig. 15a ein aus dem Stand der Technik beispielsweise gemäß der US-Patentschrift Nr. 5,725,552 bekanntes Röhrengeflecht als Ausgangsgerüst bzw. Grundkörper zur Herstellung eines solchen bekannten Occlusionsinstruments gezeigt, wobei die Enden des Röhrengeflechts jeweils mit einer Fassung gehalten werden müssen. Fig. 15b stellt eine Seitenansicht eines beispielsweise aus der US-Patentschrift Nr. 5,725,552 bekannten Occlusionsinstruments vom PFO-Typ mit einer Seitenschnittdarstellung auf der rechten Seite dar, wobei das Occlusionsinstruments aus einem Röhrengeflecht gemäß Fig. 15a hergestellt ist, während Fig. 15c eine Seitenansicht eines ebenfalls beispielsweise aus der US-Patentschrift Nr. 5,725,552 bekannten weiteren Occlusionsinstruments vom ASD-Typ mit einer Seitenschnittdarstellung auf der rechten Seite zeigt, wobei dieses Occlusionsinstruments ebenfalls aus einem Röhrengeflecht gemäß Fig. 15a hergestellt ist.

CN 2524710 offenbart einen Okkluder hergestellt durch Stricken eines einzelnen Drahtes auf eine Form. Das Okkluder enthält eine Membranbarriere innerhalb des Okkluders, und zwei Scheiben, wo die erste Scheibe eine Verbindungseinheit enthält. Die zweite Scheibe wird durch eine spezielle Stricktechnik hergestellt und hat ein spezielles Drähtmuster, die sich von dem Drahtmuster der ersten Platte differenziert.

Unter dem hierin verwendeten Begriff "PFO-Typ" ist ein Occlusionsinstrument zur Behandlung eines Patent-Foramen-Ovale (PFO) zu verstehen, während unter dem Begriff "Occlusionsinstrument vom ASD-Typ" ein Occlusionsinstrument zur Behandlung eines Vorhof-Septum-Defekts (ASD) zu verstehen ist.

Die bekannten Occlusionsinstrumente bestehen aus einem Geflecht aus einer Vielzahl feiner, geflochtener Nitinol-Drähte in Form eines Jojos. Jenes Geflecht wird in seiner ursprünglichen Form als Rundgeflecht hergestellt, welches sowohl an seinem Anfang (bzw. an seiner proximalen Seite) als auch an seinem Ende (bzw. an seiner distalen Seite) lose Drahtenden aufweist. Bei der Weiterverarbeitung des Rundgeflechtes müssen dann diese losen Enden jeweils in einer Hülse gefasst und verschweißt werden. Nach dieser entsprechenden Weiterverarbeitung weist sowohl die proximale Seite als auch die distale Seite des fertigen Occluders jeweils eine abstehende Hülse auf. In das distale und proximale Retentionsschirmchen und in dem dazwischen angeordneten Steg sind Dacron-Patches eingenäht. Aufgrund des Memory-Effektes des verwendeten Nitinol-Materials entfalten sich die beiden Retentionsschirmchen beim Verlassen des Katheders selbstständig. Dies erfolgt zunächst über eine ballonartige Zwischenstufe, wobei die RetentionsSchirmchen letztendlich beiderseits des Septums entgültig platziert eine mehr oder weniger abgeplattete Form einnehmen. Der Steg zentriert sich während des Aufspannens der Schirmchen in dem zu verschließenden Shunt selbstständig.

Durch diese beim proximalen Retentionsbereich des Occluders hervorstehende Hülse tritt das Problem auf, dass das eingesetzte Implantat emboliebedingte Probleme, insbesondere die konsekutive Embolisation, hervorruft. Dadurch, dass Anteile des Occlusionsinstruments über die Septumwand hervorragen und im ständigen Blutkontakt stehen, werden des weiteren häufig Abwehrreaktion hervorgerufen. Ferner wird oft auch eine vollständige Endothelialisierung des Verschluss-Implantats verhindert.

Des weiteren ist aus der WO 2005/020822 A1 ein Occlusionsinstrument der eingangs genannten Art sowie ein Verfahren zur Herstellung eines derartigen Occlusionsinstruments bekannt. Das darin beschriebene Occlusionsinstrument besteht im wesentlichen aus einem Geflecht dünner Drähte oder Fäden aus einem Material mit FormgedächtnisFunktion. Im expandierten Zustand weist das bekannte Occlusionsinstrument einen proximalen und einen distalen Retentionsbereich sowie einen dort zwischen angeordneten zylindrischen Steg auf.

Dadurch, dass bei diesem Stand der Technik der proximale Retentionsbereich des Geflechts eine zum proximalen Ende hin offene Form aufweist, kann erreicht werden, dass im eingesetzten Zustand des Occlusionsinstruments grundsätzlich der Randsaum des proximalen Retentionsbereiches flach an der Septumwand anliegt und der Retentionsbereich nicht über die Septumwand herausragt.

Bei dem Herstellungsverfahren gemäß der WO 2005/020822 A1 kommt eine Flechttechnik zum Einsatz, bei der ein nach oben offenes röhrenförmiges Geflecht hergestellt wird, welches nur an einem Ende mit einer Fassung zum Bündeln der Fäden bzw. Drähte des Geflechts versehen werden muss, während an der gegenüberliegenden Seite die Fäden bzw. Drähte des Geflechtes aus deren Mitte heraus untereinander verflochten werden.

Dadurch ist es möglich, ein Geflecht herzustellen, welches als Ausgangsgerüst für das bekannte Occlusionsinstrument dient, wobei der proximale Retentionsbereich des Ausgangsgerüsts eine zum proximalen Ende hin offene Form aufweist.

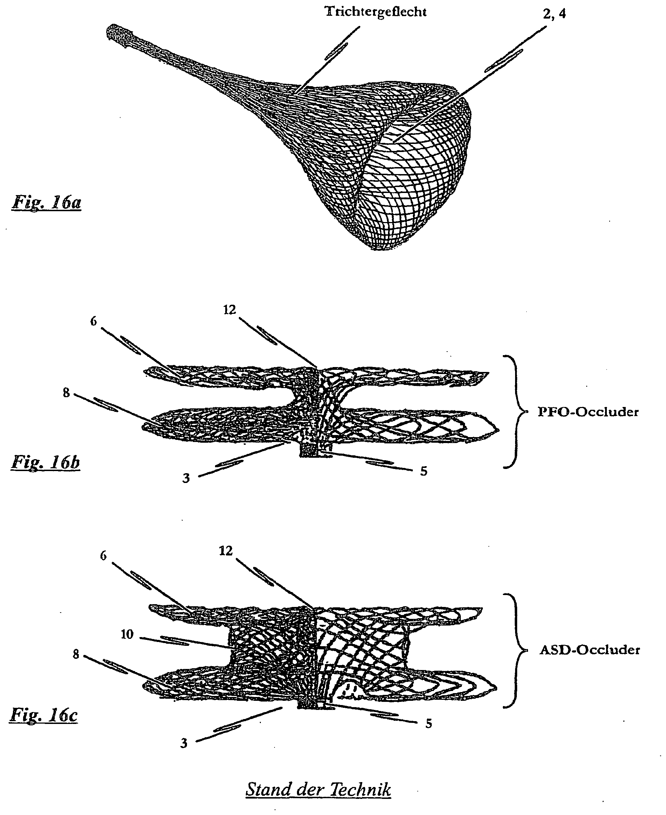

Zur näheren Erläuterung des aus der WO 2005/020822 A1 bekannten Occlusionsinstruments sei insbesondere auch auf Fig. 16a bis Fig. 16c verwiesen. Im einzelnen ist in Fig. 16a ein beispielsweise aus der WO 2005/020822 A1 bekanntes tulpen- bzw. glockenförmiges Geflecht mit einer distalen Fassung gezeigt. In Fig. 16b ist eine Seitenansicht eines aus der WO 2005/020822 A1 bekannten Occlusionsinstruments vom PFO-Typ mit einer Seitenschnittdarstellung auf der rechten Seite gezeigt, wobei das Occlusionsinstruments aus einem Röhrengeflecht gemäß Fig. 16a hergestellt ist. In Fig. 16c ist schließlich noch eine Seitenansicht eines aus der WO 2005/020822 A1 bekannten Occlusionsinstruments vom ASD-Typ mit einer Seitenschnittdarstellung auf der rechten Seite gezeigt, wobei das Occlusionsinstruments ebenfalls aus einem Röhrengeflecht gemäß Fig. 16a hergestellt ist.

Bei diesen aus der WO 2005/020822 A1 bekannten Occlusionsinstrumenten hat es sich als nachteilig erwiesen, dass das Geflecht am proximalen Ende eine Öffnung aufweist, die mit beispielsweise einer Dacron-Einlage oder mit einem Tuch überspannt werden muss, damit das fertige Occlusionsinstrument am proximalen Ende nicht mehr offen ist. Dies bedingt einen recht aufwendigen und von daher kostenintensiven Fertigungsprozess zur Herstellung eines derartigen Occlusionsinstruments. Ferner müssen verschiedene Materialien, nämlich die Materialien des Geflechts und die Materialien der Dacron-Einlage oder des Tuches, miteinander kraftschlüssig verbunden werden. Derartige Verbindungsstellen sind im Hinblick auf eine Materialermüdung grundsätzliche Schwachstellen. Demnach besteht bei diesem bekannten Occlusionstyp die erhöhte Gefahr der Materialermüdung mit Branchenfraktur. Ferner hat es sich gezeigt, dass im Körper des Patienten ein derartiges implantiertes System über einen längeren Zeitraum Materialermüdungen und Brüche in den Verbindungspunkten zwischen den metallischen Strukturen und der Dacron-Einlage aufweisen können, was aufgrund der erheblichen mechanischen Belastung herrührt. Dies ist insbesondere dann der Fall, wenn zwischen dem Geflecht und der Einlage dauerhaft Spannungen bestehen.

Ferner ist bei den aus der WO 2005/020822 A1 bekannten Occlusionsinstrumenten mit thrombembolischen Komplikationen zu rechnen. Zwar kann bei dem bekannten System erreicht werden, dass im eingesetzten Zustand des Occlusionsinstruments der Randsaum des proximalen Retentionsbereiches flach an der Septumwand anliegt und der Retentionsbereich nicht über die Septumwand herausragt, allerdings weist das proximale Ende des bekannten Occlusionsinstruments eine Proximalwand auf, in der axial zum Steg eine herstellungsbedingte Öffnung vorgesehen ist. Auch wenn diese Öffnung - wie bereits beschrieben - mittels beispielsweise der Dacron-Einlage verschlossen wird, kann bei dem bekannten System nicht verhindert werden, dass bei dem fertigen Occlusionsinstrument beim proximalen Retentionsbereich des Occluders, und zwar dort, wo die mittels der Dacron-Einlage verschlossenen Öffnung angeordnet ist, zumindest eine muldenförmige Vertiefung verbleibt oder unter Umständen auch Komponenten hervorstehen.

Mit der muldenförmigen Vertiefung und den hervorstehenden Komponenten tritt allerdings das Problem auf, dass das eingesetzte Implantat emboliebedingte Probleme, insbesondere die konsekutive Embolisation, hervorruft. Diese emboliebedingten Probleme treten insbesondere dann in Erscheinung, wenn der Patient unter einem so genannten Vorhofflimmern des Herzens leidet. Hierbei handelt es sich aus einer frequenten Erregung der Vorhöfe des Herzens, die zu keiner Kontraktion der Vorhöfe führt. Folge dieses Kontraktionsverlustes der Vorkammern des Herzens ist es, dass eine wirksame Durchwirbelung und Durchmischung des Blutes ausbleibt und sich Tromben im Vorhof bilden können. Ein erhebliches Risiko bei der Vorhoftrombenbildung infolge Vorhofflimmerns besteht darin, dass solche Tromben mit dem Blutstrom mitgerissen werden können und in die arterielle Zirkulation gelangen. Folgen dieser Embolisation sind insbesondere Schlaganfälle, die in etwa 5% pro Jahr bei Patienten mit Vorhofflimmern auftreten, falls nicht durch eine chronische Behandlung eine Geringungshemmung des Blutes mit so genannten Dicumerolen durchgeführt wird. Eine Herbeiführung der Geringungshemmung des Blutes mit so genannten Dicumerolen ist allerdings ebenfalls nicht risikolos. Nebenwirkungen der Behandlungen mit Dicumerolen sind vermehrte Blutungen, sodass Kontraindikationen für diese Behandlung bei ca. 20% der Patienten mit Vorhofflimmern besteht und die Patienten somit aufgrund von einer Risikoabwägung Blutung/Schlaganfall das Risiko eines Schlaganfalls in Kauf genommen wird.

Der vorliegenden Erfindung liegt von daher die Problemstellung zugrunde, ein solches aus der Medizintechnik bekanntes und in der WO 2005/020822 A1 beschriebenes Occlusionsinstrument, derart weiterzuentwickeln, dass die vorstehend genannten Nachteile überwunden werden können. Insbesondere soll ein Occlusionsinstrument angegeben werden, dass zum Verschluss von Defekten unterschiedlicher Größe anwendbar ist, wobei die Implantation des Occluders auf einfache Weise erfolgen kann. Ferner soll bei dem Occlusionsinstrument das Auftreten üblicher Komplikationen, wie Dislokation, Teil-Embolisation oder Materialermüdung des Verschluss-Systems, möglichst reduziert werden. Darüber hinaus soll ein Occlusionsinstrument angegeben werden, welches den Verschluss eines Septum-Defektes sicherstellt, wobei möglichst geringe Anteile des Occlusionsinstruments über die Septumwand hervorragen, um so die damit verbundenen und oben genannten Komplikationen zu vermeiden.

Auf der Grundlage dieser Problemstellung liegt der vorliegenden Erfindung die Aufgabe zugrunde, ausgehend von dem aus der WO 2005/020822 A1 bekannten System ein Occlusionsinstrument anzugeben, welches im eingesetzten Zustand an der proximalen Seite des Septum-Defektes möglichst flach mit dem Septum abschließt, und bei welchem Gefahr der Materialermüdung mit Branchenfraktur deutlich reduziert ist, und zwar bei geringeren Herstellungskosten. Der vorliegenden Erfindung liegt ferner das technische Problem zugrunde, ein Verfahren zum Herstellen eines derartigen Occlusionsinstruments anzugeben.

Diese Aufgabe wird bei einem Occlusionsinstrument der eingangs genannten Art erfindungsgemäß dadurch gelöst, dass der proximale Retentionsbereich des Geflechts am proximalen Ende des Occlusionsinstruments eine vollständig geschlossene Proximalwand aufweist, welche eine stetige Fläche aufweist, die das proximale Ende des Occlusionsinstruments bildet.

Das der vorliegenden Erfindung zugrunde liegende verfahrenstechnische Problem wird ferner durch ein Verfahren zur Herstellung des vorstehend genannten Occlusionsinstruments gelöst, das sich durch den Verfahrensschritt des Ausbildens eines kugel-, birnen- oder tropfenförmigen Hohlgeflechts mittels eines an sich bekannten Flechtverfahrens und durch den Verfahrensschritt des Ausformens eines proximalen Retentionsbereichs, eines distalen Retentionsbereichs am gebündelten ersten Ende und eines zwischen dem proximalen und dem distalen Retentionsbereich angeordneten zylindrischen Stegs auszeichnet. Dabei ist vorgesehen, dass das Hohlgeflecht an einem ersten, distalen Ende gebündelt wird und an einem gegenüberliegenden zweiten, proximalen Ende eine vollständig geschlossene Proximalwand mit einer stetigen Fläche aufweist.

Unter dem hierin verwendeten Begriff "Proximalwand" ist jener Abschnitt bzw. Bereich des proximalen Retentionsbereiches des Geflechtes am proximalen Ende des Occlusionsinstruments zu verstehen, der proximalseitig den Verschluss für den zu schließenden Defekt bildet.

Die Vorteile der Erfindung liegen insbesondere darin, dass ein intravaskuläres Occlusionsinstrument, insbesondere zur Behandlung von Septum-Defekten, angegeben wird, wobei sich das Verschlussinstrument für eine Zufuhr über einen Katheter zu dem zu verschließenden Defekt eignet. Dadurch, dass der proximale Retentionsbereich des Geflechts am proximalen Ende eine vollständig geschlossene Proximalwand aufweist, welche eine stetige Fläche aufweist, die das proximale Ende des Occlusionsinstruments bildet, kann zum einen in besonders vorteilhafter Weise erreicht werden, dass sich das Occlusionsinstrument - unabhängig von der Proportion des Durchmessers des zu verschließenden Defekts und unabhängig von der Stärke der Septumwand - an den Defekt in der Septumwand selbständig anpasst, und zwar derart, dass an der proximalen Seite des Defekts keine Anteile des Occlusionsinstruments über die Ebene, in welcher die Septumwand mit dem Defekt liegt, herausragt. Diese Ebene, d.h. die Ebene, in welcher die Septumwand mit dem Defekt liegt, wird bei der erfindungsgemäßen Lösung durch die vollständig geschlossene Proximalwand des Occlusionsinstruments gebildet. Zum anderen kann mit der erfindungsgemäßen Lösung erreicht werden, dass in dieser Proximalwand keinerlei Vertiefungen oder andere im mathematischen Sinne "Unstetigkeiten", wie etwa scharfe Kanten, Knicke etc., vorliegen, so dass die üblichen hiermit mit Zusammenhang stehenden Komplikationen, insbesondere im Hinblick auf emboliebedingte Probleme, nicht mehr auftreten können.

Vor allem kann erreicht werden, dass das eingesetzte Occlusionsinstrument wesentlich schneller als bei den aus dem Stand der Technik bekannten Verschluss-Systemen vollständig von körpereigenem Gewebe eingeschlossen wird. Aus der Verwendung eines aus dünnen Drähten oder Fäden aufgebauten Geflechts als Ausgangsmaterial für das erfindungsgemäße Occlusionsinstrument leitet sich der weitere Vorteil ab, dass es langfristig eine im Vergleich zu den aus dem Stand der Technik bekannten Systemen bessere mechanische Stabilität aufweist. Somit kann das Auftreten von Brüchen in der Struktur des eingesetzten Implantats weitgehend verhindert werden. Ferner besitzt das Geflecht eine bessere Steifigkeit, da die gesamte Struktur aus einem Material und ohne Verbindungsstellen gebildet ist.

Insbesondere dadurch, dass bei der erfindungsgemäßen Lösung vollständig auf Gewebeeinlagen bzw. Dacron-Einlagen verzichtet werden kann, wie es beispielsweise bei dem Verschlusssystem gemäß der WO 2005/020822 A1 der Fall ist, kann wirkungsvoll das frühzeitige Auftreten von Materialermüdungserscheinungen weiter reduziert werden, wobei sogar insgesamt die Herstellungskosten noch herabgesetzt werden.

Die am proximalen Retentionsbereich des Geflechtes vorgesehene vollständig geschlossene Proximalwand gestattet es zusätzlich, dass der proximale Retentionsbereich des Instruments im eingesetzten Zustand an dem Randsaum des Defektes vollständig abflacht, und zwar nahezu unabhängig von dem Durchmesser des Defektes und der Stärke der Septumwand. Demnach kann das Occlusionsinstrument über einen weiten Bereich unterschiedlich großer Septum-Defekte eingesetzt werden. Dadurch, dass am proximalen Retentionsbereich auf eine Fassung zum Zusammenbündeln bzw. Zusammenfassen des Geflechtes verzichtet werden kann, ragt auch keine Komponente des Occlusionsinstruments über die Septumwand hinaus, so dass ein ständiger Blutkontakt mit Komponenten des Implantats verhindert werden kann. Dies hat den Vorteil, dass Abwehrreaktionen des Körpers und keine thrombembolischen Komplikationen zu befürchten sind.

Mit dem erfindungsgemäßen Verfahren wird eine besonders leicht zu realisierende Möglichkeit zur Herstellung des vorstehend beschriebenen Occlusionsinstruments angegeben. Dabei wird zunächst ein kugel-, birnen-oder tropfenförmiges Hohlgeflecht mittels beispielsweise einer Rundflechtmaschine ausgebildet. Es kommt hier eine Technik zum Einsatz, bei der das ausgebildete Geflecht am Ende der Flechtlänge, d.h. am späteren distalen Ende des Occlusionsinstruments, gebündelt wird und am Anfang der Flechtlänge, d.h. am späteren proximalen Ende des Occlusionsinstruments, geschlossen ist. Damit ist es möglich, ein "sackförmiges" Hohlgeflecht herzustellen, dessen gebündeltes Ende dem distalen Ende des fertigen Occlusionsinstruments und dessen gegenüberliegendes geschlossenes Ende dem proximalen Ende bzw. der Proximalwand des fertigen Occlusionsinstruments entspricht. Dadurch, dass zum Herstellen des Occlusionsinstruments ein an sich bekanntes Flechtverfahren verwendet wird, weist das gefertigte Occlusionsinstrument mechanische Eigenschaften hinsichtlich beispielsweise der Dehnung, Stabilität, Festigkeit etc. auf, die individuell an den späteren Einsatz des Occlusionsinstruments angepasst werden können. In vorteilhafter Weise können metallische Drähte, aber auch organische Fäden zu dem Geflecht verarbeitet werden. Selbstverständlich ist unter den hierin verwendeten Begriffen "kugelförmig", "birnenförmig", "tropfenförmig" und "sackförmig" jeweils solche Formgebungen zu verstehen, die einer Kugel, einer Birne, einem Tropfen oder einem Sack ähnlich sind. Die Erfindung ist insbesondere nicht auf eine exakte Kugelform etc. beschränkt.

Bevorzugte Weiterbildungen der Erfindung sind bezüglich des Occlusionsinstruments in den Unteransprüchen 2 bis 12 und bezüglich des Herstellungsverfahrens in den Ansprüchen 13 und 14 angegeben.

Besonders bevorzugt ist vorgesehen, dass die Proximalwand des Occlusionsinstruments als stetige Fläche eine gekrümmte Fläche aufweist. Hierbei ist wesentlich, dass die Krümmung dieser Fläche keine Unstetigkeiten, wie beispielsweise Kanten, Ecken, etc., aufweist. Hinsichtlich des hierin verwendeten Begriffes "Stetigkeit" sei auf die mathematische Definition einer stetigen Fläche, wie sie in der Topologie bekannt ist, verwiesen. Denkbar wäre beispielsweise, dass die Proximalwand eine gekrümmte Fläche aufweist, die im Hinblick auf die Ebene, in welcher die Septumwand mit dem Defekt liegt, vorzugsweise leicht konkav gekrümmt ist, um somit ein besonders gutes, d.h. flaches und ebenes Anliegen der Proximalwand an der Septumwand zu gewährleisten. Selbstverständlich ist aber auch denkbar, dass die Fläche der Proximalwand im Hinblick auf die Septumwand vorzugsweise leicht konvex ausgebildet ist, wodurch erreicht werden kann, dass die kraftschlüssige Verbindung zwischen der Proximalwand bzw. dem peripheralen Bereich der Proximalwand mit der Septumwand am zu verschließenden Defekt besonders groß ist, um somit ein besseres Fixieren des Occlusionsinstruments im zu schließenden Defekt zu ermöglichen. Auch wäre es denkbar, dass die Proximalwand am Außenbereich beispielsweise leicht konkav geformt ist, während sie in Richtung Mitte, d.h. zur Position, die axial zum Steg des Occlusionsinstruments liegt, in eine konvexe Formgebung übergeht. Auch dies kann insbesondere im Hinblick auf die Fixierung des Occlusionsinstruments im zu verschließenden Defekt Vorteile mit sich ziehen.

In einer besonders bevorzugten Realisierung der zuletzt genannten Ausführungsform, bei welche die Proximalwand des Occlusionsinstruments als stetige Fläche eine gekrümmte Fläche aufweist, ist vorgesehen, dass die gekrümmte Fläche mit der Oberfläche eines Abschnitts eines kugel-, birnen- oder tropfenähnlichen Körpers übereinstimmt. Anders ausgedrückt bedeutet dies, dass die Proximalwand des Occlusionsinstruments beispielsweise in Gestalt einer Kugelkappe oder eines Tropfensegments gebildet sein kann. Selbstverständlich sind hier aber auch andere Formgebungen denkbar. Vorteilhaft ist insbesondere, dass mit der erfindungsgemäßen Lösung ein Occlusionsinstrument angegeben werden kann, welches unabhängig von der Art und insbesondere auch der Größe des zu verschließenden Defekts grundsätzlich optimal angewandt werden kann.

Insbesondere ist für das Occlusionsinstrument bevorzugt vorgesehen, dass das Geflecht aus Nitinol oder aus einem anderen Material mit Formgedächtnis- oder Memory-Effekt besteht. Als andere Materialien könnten beispielsweise Kupfer-Zink-AluminiumLegierungen, Gold-Cadmium-Legierungen oder auch Legierungen auf einer Eisen-Basis, wie z.B. Eisen-Mangan-Silicium-Legierungen, aber auch Kunststoffe in Frage kommen, die allesamt dadurch gekennzeichnet sind, dass sie über ein extrem hohes Erinnerungsvermögen verfügen.

Für das erfindungsgemäße Occlusionsinstrument ist insbesondere vorgesehen, dass das Geflecht aus einem Formgedächtnispolymer ausgebildet ist, welches beispielsweise auf Polyanhydriden als Matrix oder auf Polyhydroxycarbonsäuren basieren. Hierbei handelt es sich um synthetische, abbaubare Materialien, die über einen thermisch induzierten Formgedächtniseffekt verfügen. Denkbar wären aber auch andere Formgedächtnispolymere, wie etwa Blockcopolymere, wie sie beispielsweise im Sonderdruck Angewandte Chemie 2002, 114, Seiten 2138 bis 2162, von A. Lendlein und S. Kelch beschrieben werden.

Durch die Verwendung eines derartigen Materials ist es möglich, als Ausgangskörper für das Occlusionsinstrument ein beispielsweise mittels eines Rundflechtverfahrens erzeugtes, sackförmiges Hohlgeflecht zu verwenden, welches an seinem einen Ende geschlossen und an seinem anderen Ende offen und gebündelt ist. Jener Ausgangskörper wird anschließend mittels eines Umformungs- und Wärmebehandlungsverfahrens in die gewünschte Form des Occlusionsinstruments gebracht. Selbstverständlich sind hier aber auch andere Weiterverarbeitungsverfahren denkbar.

In einer vorteilhaften Weiterentwicklung der zuletzt genannten Ausführungsform des erfindungsgemäßen Occlusionsinstruments, bei welchem das Geflecht aus einem Formgedächtnismaterial gebildet wird, ist vorgesehen, dass das Material ein biologisch abbaubares Formgedächtnis-Polymermaterial aufweist. Insbesondere eignen sich synthetische, bioabbaubare Implantatmaterialen. Derartige abbaubare Werkstoffe bzw. Polymere enthalten unter physiologischen Bedingungen spaltbare Bindungen. Dabei spricht man von einer "Bioabbaubarkeit", wenn der Werkstoff unter Verlust der mechanischen Eigenschaft durch oder in einem biologischen System abgebaut wird. Die äußere Form sowie die Maße des Implantats bleiben während des Abbaus unter Umständen erhalten. Wird von einer Degradationszeit ohne zusätzliche quantifizierende Angabe gesprochen, so ist die Zeit, in der der vollständige Verlust der mechanischen Eigenschaft auftritt, gemeint. Unter biostabilen Werkstoffen versteht man solche, die in biologischen Systemen stabil sind und langfristig zumindest dort teilweise abgebaut werden.

Bei abbaubaren Polymeren unterscheidet man zwischen hydrolytisch und enzymatisch abbaubare Polymere. Der hydrolytische Abbau hat den Vorteil, dass die Abbaugeschwindigkeit unabhängig vom Ort der Implantation ist, da Wasser überall vorhanden ist. Dem gegenüber ist die Konzentration an Enzymen lokal sehr unterschiedlich. Bei bioabbaubaren Polymeren oder Werkstoffen kann demnach der Abbau durch reine Hydrolyse, enzymatisch induzierte Reaktionen oder durch deren Kombination erfolgen. Typische hydrolysierbare chemische Bindungen sind Amid-, Esther- oder Acethal-Bindungen. Bei dem Abbau beobachtet man zwei Mechanismen. Bei einem Oberflächenabbau findet die Hydrolyse chemischer Bindungen ausschließlich an der Oberfläche statt. Aufgrund des hydrophoben Charakters erfolgt der Polymerabbau schneller als die Diffusion von Wasser in das Innere des Materials. Diesen Mechanismus beobachtet man vor allem bei Poly(anhydride)n oder Poly(ortoesther)n. Für die vor allem für den Formgedächtnis-Effekt bedeutsamen Poly(hydroxcarbonsäuren), wie etwa Poly(milchsäure) oder Poly(glykosesäure) bzw. entsprechend Copolymere, erfolgt der Polymerabbau im gesamten Volumen. Der geschwindigkeitsbestimmende Schritt ist hierbei die hydrolytische Bindungsspaltung, da die Diffusion von Wasser in der eher hydrophilen Polymermatrix relativ schnell erfolgt. Für die Anwendung von bioabbaubaren Polymeren ist entscheidend, dass sie sich einerseits mit einer kontrollierbaren bzw. einstellbaren Geschwindigkeit abbauen, und andererseits die Abbauprodukte nicht toxisch sind.

Besonders bevorzugt ist bei einer Weiterbildung des erfindungsgemäßen Occlusionsinstruments vorgesehen, dass sich das Geflecht des Occlusionsinstruments auf den Durchmesser eines bei dem minimal-invasiven Operationseingriff verwendeten Katheters verjüngen lässt. Der Vorteil dieser Ausführungsform ist insbesondere darin zu sehen, dass die zur Implantation und Explantation zu verwendenden Kathetersysteme einen deutlich reduzierten Innendurchmesser aufweisen können, was vor allem die Manövrierfähigkeit des zu implantierenden Occlusionsinstruments deutlich erhöht. Von daher kann die Positioniergenauigkeit des Instruments im zu verschließenden Defekt verbessert werden. Bei einem aus Nitinol bestehenden Occluder liegt der Innendurchmesser des zur Implantation oder Explantation verwendeten Katheters zwischen 8 bis 10 frenches, wohingegen bei der Verwendung von Occlusionsinstrumenten aus Polymerkunststoff der Innendurchmesser lediglich zwischen 6 bis 8 frenches liegen muss.

Im Hinblick auf die zuletzt genannte bevorzugte Ausführungsform der erfindungsgemäßen Lösung, wonach sich das Geflecht des Occlusionsinstruments auf den Durchmesser eines bei dem intravaskulären Operationseingriff verwendeten Katheters verjüngen lässt, ist bei einer Weiterentwicklung vorgesehen, dass der proximale Retentionsbereich des Occlusionsinstruments mit seiner Proximalwand derart ausgelegt ist, dass sich die Proximalwand beim Expandieren des Occlusionsinstruments nach außen wölbt, um derart mit der Septumwand zur Anlage zu kommen. Hierbei handelt es sich um eine besonders leicht zu realisierende und dabei wirkungsvolle Weise, die Proximalwand beim Occlusionsinstrument auszubilden. Insbesondere ist es somit möglich, das gesamte Occlusionsinstrument aus einem einstückigen Geflecht zu bilden, so dass einerseits keine mechanischen Verbindungselemente zwischen der Proximalwand und dem Steg notwendig sind, und andererseits die Dimension des Occlusionsinstruments im zusammengefalteten Zustand weiter minimiert werden kann. Selbstverständlich sind hier aber auch andere Ausführungsformen zum Ausbilden der Proximalwand am proximalen Retentionsbereich denkbar.

Damit das erfindungsgemäße Occlusionsinstrument die Funktionalität der Rückhohlbarkeit aufweist, ist bei einer bevorzugten Weiterentwicklung des Occlusionsinstruments vorgesehen, dass der distale Retentionsbereich, vorzugsweise axial zum Steg angeordnet, die eine Fassung aufweist, wobei die Fassung zumindest ein Verbindungselement aufweist, welches mit einem Katheter in Eingriff bringbar ist. Mit diesem Verbindungselement, welches in bevorzugter Weise am distalen Ende des Occlusionsinstruments derart angeordnet ist, dass es nicht über die distalseitige Septumwand hinausragt, wodurch ein ständiger Blutkontakt mit Komponenten des Implantats verhindert werden kann, kann das Occlusionsinstrument gemäß dieser Weiterentwicklung auf einfache Weise wieder explantiert werden. Andererseits erleichtert ein Verbindungselement, welches mit einem Katheter in Eingriff bringbar ist, das Implantieren und Positionieren des (beim Implantationsvorgang zusammengefalteten) Occlusionsinstrument in dem zu verschließenden Septum-Defekt. Als Verbindungselement kommen unterschiedliche Einrichtungen in Frage. Denkbar wären beispielsweise Einrastglieder oder auch Haken bzw. Ösen, die mit entsprechend komplementär ausgebildeten Verbindungselementen eines Katheters kraftschlüssig verbunden werden können.

In einer weiteren vorteilhaften Weiterentwicklung ist vorgesehen, dass das Occlusionsinstrument reversibel zusammen- und auseinanderfaltbar ausgeführt ist, so dass das Instrument in seinem expandierten Zustand, beispielsweise mit Hilfe eines Explantationskatheters zusammenfaltbar ist, wobei die kraftschlüssige Verbindung zwischen der am proximalen Retentionsbereich gebildeten Proximalwand bzw. dem peripheren Bereich der Proximalwand und der Septumwand gelöst wird. Dabei ist denkbar, dass zur Explantation ein Katheter beispielsweise an einem am distalen Ende des Occlusionsinstruments gebildeten Verbindungselement eingreift und durch eine externe Manipulation mit Hilfe des Katheters das Zusammenfalten des Occlusionsinstruments bewirkt wird. Somit ist das Occlusionsinstrument vollkommen reversibel in den Katheter zurückziehbar, welches das vollständige Entfernen des Instruments ermöglicht.

Mit dem erfindungsgemäßen Verfahren wird eine besonders leicht zu realisierende Möglichkeit zur Herstellung des vorstehend beschriebenen Occlusionsinstruments angegeben. Dabei wird zunächst ein kugelförmiges bzw. sackförmiges Hohlgeflecht mittels beispielsweise einer Rundflechtmaschine ausgebildet, wie es bereits in der Patentanmeldung WO 2005/020822 A1 beschrieben ist. Allerdings ist hierzu ein spezieller Flechtkopf notwendig, welcher nachfolgend unter Bezugnahme auf die Figuren näher erläutert wird. Hier kommt insbesondere eine Technik zum Einsatz, bei der das ausgebildete Geflecht am Ende der Flechtlänge, d.h. am späteren distalen Ende des Occlusionsinstruments, gebündelt wird und am Anfang der Flechtlänge, d.h. am späteren proximalen Ende des Occlusionsinstruments, geschlossen bleibt. Damit ist es möglich, ein kugelähnliches bzw. sackformähnliches Hohlgeflecht herzustellen, dessen gebündeltes Ende dem distalen Ende des fertigen Occlusionsinstruments und dessen gegenüberliegendes geschlossenes Ende dem proximalen Ende des fertigen Occlusionsinstruments entspricht. Das gefertigte Occlusionsinstrument weist mechanische Eigenschaften hinsichtlich beispielsweise der Dehnung, Stabilität, Festigkeit etc. auf, die individuell an den späteren Einsatz des Occlusionsinstruments angepasst werden können. In vorteilhafter Weise können metallische Drähte, aber auch organische Fäden zu dem Geflecht verarbeitet werden.

Hinsichtlich des Verfahrens ist vorgesehen, dass der Verfahrensschritt des Ausformens der Retentionsbereiche und des Stegs einen Umformungs- und Wärmebehandlungsschritt aufweist. Dieses ist insbesondere dann von Vorteil, wenn das ausgebildete kugelförmige Hohlgeflecht aus Nitinol oder aus einem anderen Material mit Formgedächtnis- oder Memory-Effekt besteht. Für das erfindungsgemäße Occlusionsinstrument ist insbesondere vorgesehen, dass das Geflecht aus einem Formgedächtnispolymer ausgebildet ist, welches beispielsweise auf Polyanhydriden als Matrix oder auf Polyhydroxidcarbonsäuren basieren. Hierbei handelt es sich um synthetische, abbaubare Materialien, die über einer thermisch induzierten Formgedächtniseffekt verfügen. Denkbar wären aber auch andere Formgedächtnispolymere, wie etwa Blockcopolymere. Wesentlich ist, dass derartige Materialien sich auf einfache Weise durch eine Kombination aus Umformungs- und Wärmebehandlungsschritten in eine entsprechende endgültige Form bringen lassen. Ein fertig ausgebildeter Occluder lässt sich dann beispielsweise auf die Größe eines Katheters verjüngen. Nach Austritt aus dem Katheter entfaltet sich das Occlusionsinstrument dann selbständig und nimmt wieder jene Formgebung an, die mittels des Umformungs- und/oder Wärmebehandlungsschritts dem kugelförmigen Hohlgeflecht des Occlusionsinstruments beim Herstellungsverfahren aufgeprägt wurde. Das kugelförmige Hohlgeflecht ist derart hergestellt, dass Drähte, die das fertige Geflecht begründen, beim Ausbilden des kugelförmigen Hohlgeflechts am proximalen Ende des Geflechts untereinander verflochten werden. Dies stellt eine mögliche und besonders einfach zu realisierende Art und Weise dar, ein Occlusionsinstrument gemäß der vorliegenden Erfindung herzustellen, dessen proximaler Retentionsbereich eine zum proximalen Ende hin geschlossene, flache Form aufweist (Proximalfläche).

Im folgenden werden bevorzugte Ausführungsbeispiele des erfindungsgemäßen Occlusionsinstruments sowie eine Rundflechtmaschine zur beispielhaften Erläuterung des erfindungsgemäßen Herstellungsverfahrens des Occlusionsinstruments anhand der Zeichnungen näher erläutert.

Es zeigen:
- Fig. 1: eine perspektivische Ansicht einer bevorzugten ersten Ausführungsform eines erfindungsgemäßen Occlusionsinstruments im expandierten Zustand, bei der nur die Umrisse des Occlusionsinstruments dargestellt sind;
- Fig. 2: eine perspektivische Detailansicht auf den distalen Retentionsbereich der ersten Ausführungsform des in Fig. 1 gezeigten Occlusionsinstruments im expandierten Zustand;
- Fig. 3: eine Seitenansicht des in Fig. 2 gezeigten erfindungsgemäßen Occlusionsinstruments im expandierten Zustand;
- Fig. 4: eine dreidimensionale Ansicht einer Rundflechtmaschine zum Erläutern des erfindungsgemäßen Herstellungsverfahrens des Occlusionsinstruments;
- Fig. 5: eine Draufsicht auf die in der Fig. 4 dargestellte Rundflechtmaschine zum Erläutern des erfindungsgemäßen Herstellungsverfahrens eines kugelförmigen, birnenförmigen oder tropfenartigen Grundgeflechts gemäß Fig. 9a bis Fig. 9c, welches als Ausgangsgerüst für das erfindungsgemäße Occlusionsinstrument dienen kann;
- Fig. 6: einen Flechtkopf der in Fig. 4 dargestellten Rundflechtmaschine im Detail;
- Fig. 7: ein Beispiel eines mittels des in Fig. 6 gezeigten Flechtkopfs hergestellten Geflechts, welches als Ausgangsgerüst für das erfindungsgemäße Occlusionsinstrument dienen kann;
- Fig. 8a: eine Seitenansicht eines speziellen Flechtkopfes zur Herstellung eines kugelförmigen, birnenförmigen oder tropfenartigen Grundgeflechts gemäß Fig. 9a bis Fig. 9c, welches als Ausgangsgerüst für das erfindungsgemäße Occlusionsinstrument dienen kann;
- Fig. 8b: eine Schnittdarstellung des Flechtkopfes gemäß Fig. 8a;
- Fig. 8c: eine räumliche Darstellung des speziellen Flechtkopfes zur Herstellung eines Kugelgeflechtes;
- Fig. 9a: eine perspektivische Darstellung eines Grundkörpers eines Kugelgeflechtes als Ausgangskörper für das erfindungsgemäße Occlusionsinstrument, wobei der Grundkörper weitestgehend als Kugel ausgebildet ist;
- Fig. 9b: eine perspektivische Darstellung eines Grundkörpers eines Kugelgeflechtes, welches zur Herstellung des erfindungsgemäßen Occlusionsinstruments geeignet ist, wobei der Grundkörper weitestgehend in der Form eines birnenförmigen Körpers gebildet ist;
- Fig. 9c: eine perspektivische Darstellung eines Grundkörpers eines Kugelgeflechtes, wobei der Grundkörper zur Herstellung des erfindungsgemäßen Occlusionsinstruments geeignet ist, und wobei der Grundkörper in Form eines tropfenförmigen Gebildes ausgebildet ist;
- Fig. 10a: ein Kugelgeflecht als Grundkörper für verschiedene Occlusionsinstrumente gemäß der vorliegenden Erfindung, welches nach einer speziellen Flechtmethode hergestellt ist, und welches eine distale Fassung aufweist;
- Fig. 10b: eine Seitenansicht eines erfindungsgemäßen Occlusionsinstruments vom PFO-Typ mit einer Seitenschnittdarstellung auf der rechten Seite, wobei das Occlusionsinstruments aus einem Kugelgeflecht gemäß Fig. 9a hergestellt ist;
- Fig. 10c: eine Seitenansicht eines erfindungsgemäßen Occlusionsinstruments vom ASD-Typ mit einer Seitenschnittdarstellung auf der rechten Seite, wobei das Occlusionsinstruments aus einem Kugelgeflecht gemäß Fig. 9a hergestellt ist;
- Fig. 11: eine Seitenansicht eines erfindungsgemäßen Occlusionsinstruments vom PFO-Typ mit einer Seitenschnittdarstellung auf der rechten Seite, wobei das Occlusionsinstruments aus einem kugelförmigen, birnenförmigen oder tropfenartigen Grundgeflecht gemäß Fig. 9a bis Fig. 9c mit einer distalen Fassung hergestellt ist;
- Fig. 12: eine Seitenansicht eines erfindungsgemäßen Occlusionsinstruments vom VSD-Typ mit einer Seitenschnittdarstellung auf der rechten Seite, wobei das Occlusionsinstruments aus einem kugelförmigen, birnenförmigen oder tropfenartigen Grundgeflecht gemäß Fig. 9a bis Fig. 9c mit einer distalen Fassung hergestellt ist;
- Fig. 13: eine Seitenansicht eines erfindungsgemäßen Occlusionsinstruments vom ASD-Typ mit einer Seitenschnittdarstellung auf der rechten Seite, wobei das Occlusionsinstruments aus einem kugelförmigen, birnenförmigen oder tropfenartigen Grundgeflecht gemäß Fig. 9a bis Fig. 9c mit einer distalen Fassung hergestellt ist;
- Fig. 14: eine Seitenansicht eines erfindungsgemäßen Occlusionsinstruments vom PDA-Typ mit einer Seitenschnittdarstellung auf der rechten Seite, wobei das Occlusionsinstruments aus einem kugelförmigen, birnenförmigen oder tropfenartigen Grundgeflecht gemäß Fig. 9a bis Fig. 9c mit einer distalen Fassung hergestellt ist;
- Fig. 15a: ein aus dem Stand der Technik beispielsweise gemäß der US-Patentschrift Nr. 5,725,552 bekanntes Röhrengeflecht als Ausgangsgerüst bzw. Grundkörper zur Herstellung eines bekannten Occlusionsinstruments, wobei die Enden des Röhrengeflechts jeweils mit einer Fassung gehalten werden müssen;
- Fig. 15b: eine Seitenansicht eines aus dem Stand der Technik beispielsweise gemäß der US-Patentschrift Nr. 5,725,552 bekannten Occlusionsinstruments vom PFO-Typ mit einer Seitenschnittdarstellung auf der rechten Seite, wobei das Occlusionsinstruments aus einem Röhrengeflecht gemäß Fig. 15a hergestellt ist;
- Fig. 15c: eine Seitenansicht eines aus dem Stand der Technik beispielsweise gemäß der US-Patentschrift Nr. 5,725,552 bekannten Occlusionsinstruments vom ASD-Typ mit einer Seitenschnittdarstellung auf der rechten Seite, wobei das Occlusionsinstruments aus einem Röhrengeflecht gemäß Fig. 15a hergestellt ist;
- Fig. 16a: ein aus dem Stand der Technik gemäß beispielsweise der WO 2005/020822 A1 bekanntes tulpen- bzw. glockenförmiges Geflecht mit einer distalen Fassung;
- Fig. 16b: eine Seitenansicht eines aus dem Stand der Technik gemäß beispielsweise der WO 2005/020822 A1 bekannten Occlusionsinstruments vom PFO-Typ mit einer Seitenschnittdarstellung auf der rechten Seite, wobei das Occlusionsinstruments aus einem Röhrengeflecht gemäß Fig. 16a hergestellt ist; und
- Fig. 16c: eine Seitenansicht eines aus dem Stand der Technik gemäß beispielsweise der WO 2005/020822 A1 bekannten Occlusionsinstruments vom ASD-Typ mit einer Seitenschnittdarstellung auf der rechten Seite, wobei das Occlusionsinstruments aus einem Röhrengeflecht gemäß Fig. 16a hergestellt ist.

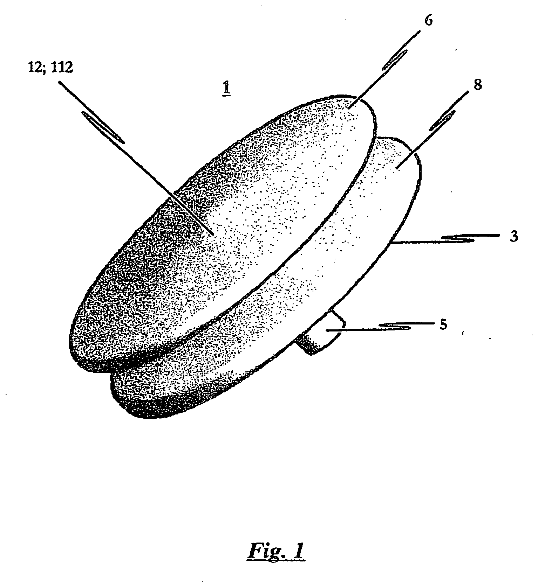

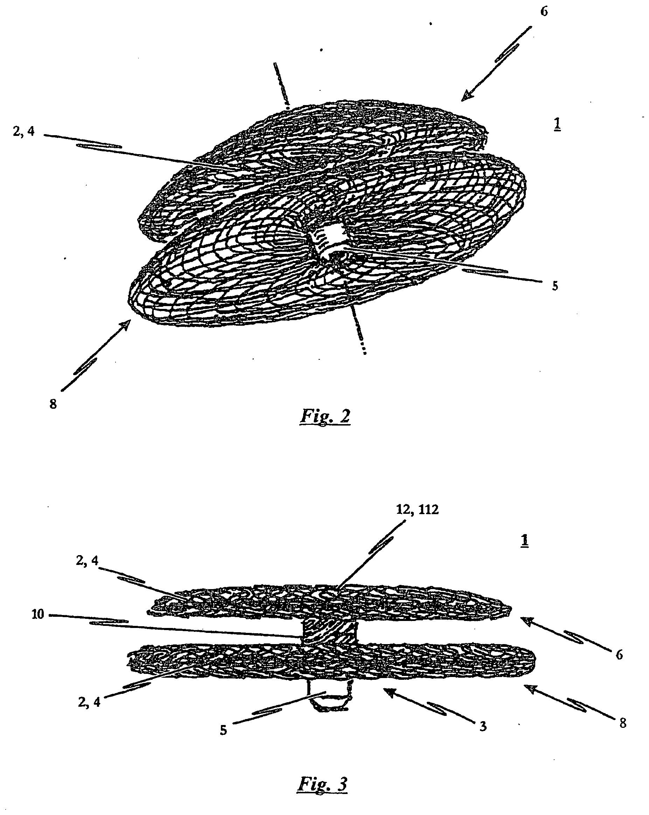

Fig. 1 zeigt eine perspektivische Ansicht einer bevorzugten ersten Ausführungsform des erfindungsgemäßen Occlusionsinstruments 1 im expandierten Zustand, wobei in Fig. 1 nur die Umrisse des Occlusionsinstruments 1 dargestellt sind. Fig. 2 zeigt eine perspektivische Detailansicht auf den distalen Retentionsbereich 8 der ersten Ausführungsform des in Fig. 1 gezeigten Occlusionsinstruments 1 im expandierten Zustand. Fig. 3 zeigt eine Seitenansicht des in Fig. 2 gezeigten erfindungsgemäßen Occlusionsinstruments 1 im expandierten Zustand.
Das Occlusionsinstrument 1 besteht im wesentlichen aus einem Geflecht 2 dünner Drähte 4, die in bevorzugter Weise aus Nitinol oder aus einem anderen Material mit Formgedächtnis- oder Memory-Effekt bestehen. Das Geflecht 2 weist eine ausreichende Flexibilität auf, so dass das Occlusionsinstrument 1 auf den Durchmesser eines bei einem intravaskulären Operationseingriff verwendeten (nicht explizit dargestellten) Katheters verjüngt werden kann. Auf Grund des Memory-Effektes des Materials verfügt das derart verjüngte Occlusionsinstrument 1 eine Formgedächtnisfunktion, so dass sich das Instrument 1 nach dem Verlassen des Katheters selbstständig expandiert und eine der Verwendung entsprechende, vorbestimmte Form wieder einnimmt. Üblicherweise erfolgt dies, nachdem das zunächst im Katheter angeordnete Occlusionsinstrument 1 an der zu behandelnden Stelle platziert wurde.

Wie insbesondere in den Figuren 2 und 3 dargestellt, weist das Occlusionsinstrument 1 im expandierten Zustand einen proximalen Retentionsbereich 6, einen distalen Retentionsbereich 8 und einen zylindrischen Steg 10 auf, der zwischen den proximalen und den distalen Retentionsbereich 6, 8 angeordnet ist. Die beiden Retentionsbereiche 6, 8 dienen dazu, einen in einem Septum vorhandenen Defekt bzw. Shunt zu verschließen. Dies erfolgt derart, dass jene Bereiche 6, 8 beiderseits an den zu verschließenden Shunt zur Anlage kommen, während der Steg 10 durch den Shunt hindurchläuft. Das erfindungsgemäße Occlusionsinstrument 1 stellt von daher ein Verschlusssystem dar, das mittels minimalinvasiver Verfahren, d.h. beispielsweise über einen Katheter und Führungsdrähte, dem Körper eines Patienten zugeführt und an der dafür bestimmten Stelle positioniert wird.

Der Konstruktion des erfindungsgemäßen Instruments 1 liegt dabei das Prinzip zu Grunde, das sich das Occlusionsinstrument 1 auf die Größe des Katheters verjüngen lässt. Nach dem Austritt aus dem Katheter entfalten sich dann die Retentionsbereiche 6, 8 selbstständig und legen sich beiderseits des Septums an. Durch die erfindungsgemäße Konstruktion handelt es sich bei dem Occlusionsinstrument 1 ferner um ein selbstpositionierendes und selbst-zentrierendes System. Der Steg 10 hat dabei die Länge der atrialen Scheidewand bzw. des Septums, um eine feste Anlage der Retentionsbereiche 6, 8 an der Septumwand zu gewährleisten.

Im Gegensatz zu den herkömmlichen, aus dem Stand der Technik bekannten Verschluss-systemen, bei denen als proximaler Retentionsbereich 6 ein selbst-expandierendes Schirmchen dient, liegt bei der vorliegenden Erfindung als proximaler Retentionsbereich 6 eine zum proximalen Ende 12 hin geschlossene flache Abdeckung in Gestalt einer Proximalwand 112 vor, so dass keinerlei Material des implantierten Occlusionsinstruments 1 über die Septumwand in den proximalen Bereich des Organs des Patienten hineinragen kann. Durch die zum proximalen Ende 12 hin geschlossene Formgebung des proximalen Retentionsbereiches 6 kann ferner erreicht werden, dass der Rand des proximalen Retentionsbereiches 6 immer bündig mit der Septumwand abschließt. Dies erfolgt über einen relativ weiten Bereich unabhängig von dem Defektdurchmesser und der Stärke der atrialen Scheidewand bzw. des Septums. Dadurch kann erreicht werden, dass nach dem Implantieren des Occlusionsinstrument 1 relativ schnell eine vollständige Endothelialisierung eintritt und mögliche Abwehrreaktionen vom Körper des Patienten nicht eintreten, da ein Blutkontakt mit dem Werkstoff des Implantats 1 wirksam verhindert wird.

Durch die selbstexpandierende Eigenschaft des Implantats 1 aufgrund des Memory-Effektes des verwendeten Materials weist das erfindungsgemäße Occlusionsinstrument 1 eine selbst-zentrierende Funktion im Shunt bzw. im Defekt des Septums auf. Ferner ist das Occlusionsinstrument 1 bis zum Abkoppeln des Führungsdrahtes des Einführbesteckes jederzeit zurückziehbar.

Das erfindungsgemäße Occlusionsinstrument 1 kann des weiteren selbstverständlich auch Gewebeeinlagen aufweisen, welche in den vorliegenden Zeichnungen nicht explizit gezeigt werden, und welche dem Prinzip nach aus dem Stand der Technik bekannt sind. Jene Gewebeeinlagen bestehen meistens aus dem Material Dacron. Dabei handelt es sich chemisch um den Polyester Polyethylenterephthalat, der technisch durch Polykondensation aus Ethylenglycol und Terephthalsäure -dimethylester hergestellt wird. Denkbar ist dabei, die Gewebeeinlagen im Inneren des Steges 10 oder am proximalen Ende 12 des Retentionsbereiches 6 einzuarbeiten, um den Defekt bzw. Shunt in der Septumwand vollständig verschließen zu können. Die Einlagerung der Gewebeeinlagen kann zum Beispiel durch Verspannung dieser im Occlusionsinstrument 1 erfolgen. Das im Körper eingesetzte Implantat 1 wird dann nach einigen Wochen bzw. Monaten vollständig von körpereigenem Gewebe eingeschlossen.

Das als Grundgerüst für das erfindungsgemäße Occlusionsinstrument 1 dienende Geflecht 2 weist eine ausreichende Steifigkeit auf, dass es die Gewebeeinlage aufspannt und in seiner Position verbleibt.

Am distalen Ende 3 des distalen Retentionsbereiches 8 ist das Geflecht 2 in einer Fassung 5 zusammengefasst. Dies erfolgt dabei derart, dass in der Fassung 5 ein Innengewinde hergestellt werden kann, was dazu dient, mit einem Führungsdraht eines nicht dargestellten Einführbesteckes in Eingriff zu stehen, während das Occlusionsinstrument 1 durch beispielsweise einen intravaskulären Operationseingriff an die entsprechende Position, wo der Defekt in dem Septum vorliegt, gebracht wird. Nach der Positionierung des Occlusionsinstruments 1 im Shunt bzw. Defekt wird der Eingriff zwischen dem Führungsdraht des Einführbesteckes und dem distalen Ende 3 wieder gelöst. Selbstverständlich ist aber auch denkbar, anstelle eines Innengewindes in der Fassung 5 des distalen Ende 3 eine anders ausgeführte Vorrichtung anzubringen.

Wie bereits angedeutet, zeigt Fig. 1 eine perspektivische Ansicht der bevorzugten ersten Ausführungsform des erfindungsgemäßen Occlusionsinstruments 1 im expandierten Zustand, während Fig. 2 eine perspektivische Teilansicht auf den distalen Retentionsbereich 8 des in Fig. 1 gezeigten Occlusionsinstruments 1 zeigt. Bei der Fig. 1 sind zur Vereinfachung nur die Umrisse des Occlusionsinstruments 1 dargestellt. Hier wird zu einer weiteren Vereinfachung auf eine detaillierte Darstellung des als Grundkörper dienenden Geflechtes 2 verzichtet, und die Formgebung des Occlusionsinstruments 1 ist in der Gestalt von geschlossenen Flächen dargestellt. Dieses Occlusionsinstrument 1 weist einen im Vergleich zur ersten Ausführungsform stärker abgeflachten proximalen Retentionsbereich 6 auf. Je nach Verwendungszweck wird der proximale Retentionsbereich 6 mehr oder weniger stark abgeflacht ausgebildet, so dass im expandierten Zustand letztendlich die Proximalwand 112 gebildet wird. Denkbar wäre hier aber auch eine Proximalwand 112, die eine völlig abgeflachte Kugelform oder eine nahezu tellerförmige Formgebung aufweist.

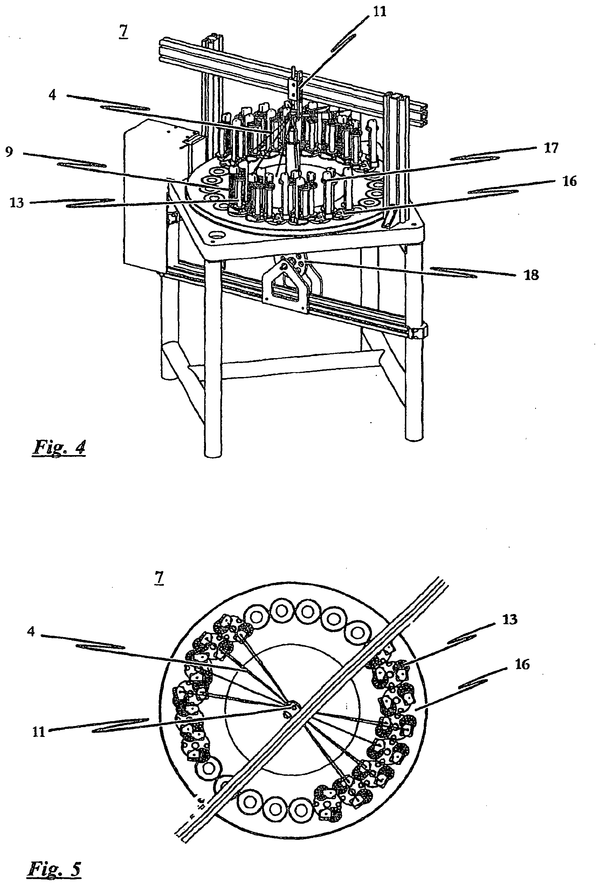

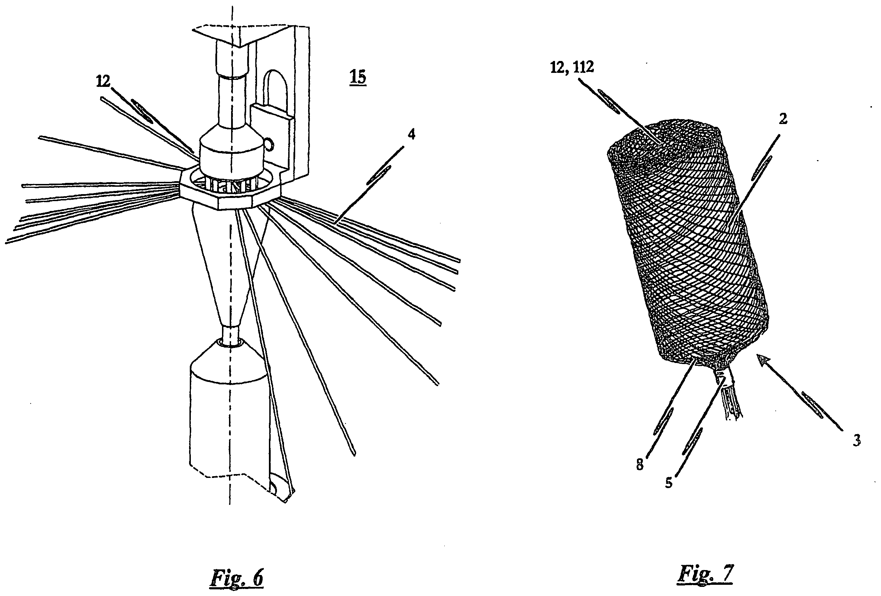

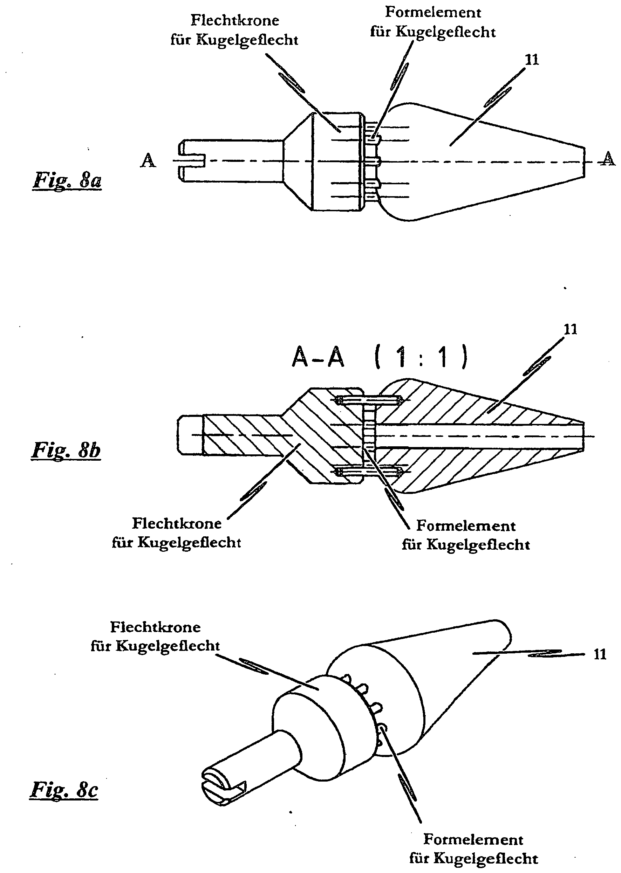

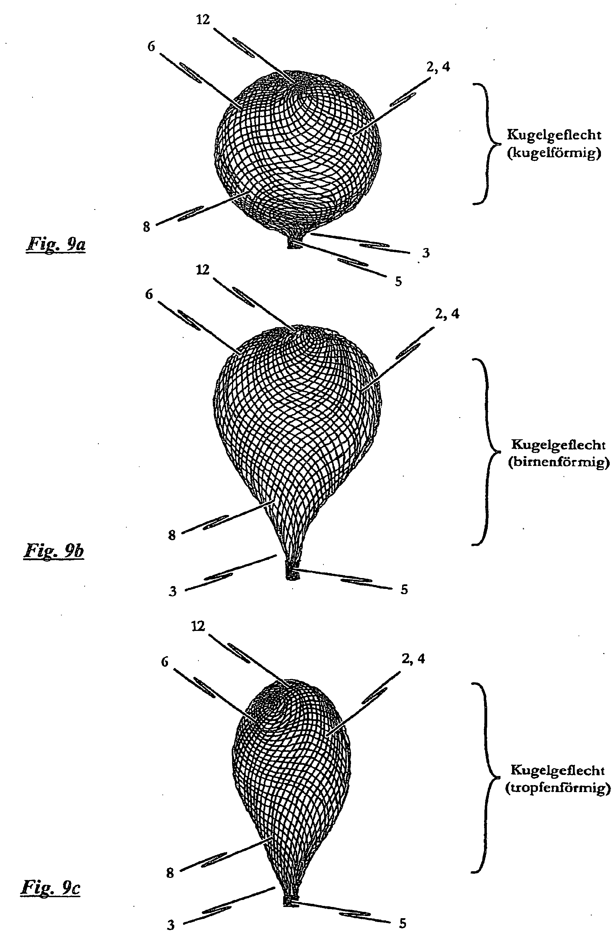

Fig. 4 zeigt eine dreidimensionale Ansicht einer Rundflechtmaschine 7 zum Erläutern des erfindungsgemäßen Herstellungsverfahrens des Occlusionsinstruments 1. In Fig. 5 ist eine Draufsicht auf die in Fig. 4 dargestellte Rundflechtmaschine 7 gezeigt, um das erfindungsgemäße Herstellungsverfahren eines kugelförmigen, birnenförmigen oder tropfenartigen Grundgeflechts 2 gemäß Fig. 9a bis Fig. 9c zu erläutern, welches aus Ausgangsgerüst für das erfindungsgemäße Occlusionsinstrument 1 dienen kann. Ferner ist in Fig. 6 ein Flechtkopf 11 der in Fig. 4 dargestellten Rundflechtmaschine 7 im Detail gezeigt, während in Fig. 7 ein Beispiel eines mittels des in Fig. 6 gezeigten Flechtkopfs 11 hergestellten Geflechts 2 dargestellt ist, welches als Ausgangsgerüst für das erfindungsgemäße Occlusionsinstrument 1 dienen kann. Des weiteren zeigt Fig. 8a eine Seitenansicht eines speziellen Flechtkopfs 11 zur Herstellung eines kugelförmigen, birnenförmigen oder tropfenartigen Grundgeflechts 2 gemäß Fig. 9a bis Fig. 9c, welches ebenfalls als Ausgangsgerüst für das erfindungsgemäße Occlusionsinstrument 1 dienen kann. Der Flechtkopf 11 gemäß Fig. 8a ist des weiteren in Fig. 8b in einer Schnittdarstellung gezeigt, während in Fig. 8c eine räumliche Darstellung des speziellen Flechtkopfes 11 zur Herstellung eines derartigen Kugelgeflechtes dargestellt ist.

Im Gegensatz zu den bekannten Flechtverfahren, wo am Anfang des Geflechts 2 alle Fäden oder Drähte 4 in einem Bündel gefasst und zu einer Abzugsvorrichtung hin gespannt werden, wird bei dem erfindungsgemäßen Verfahren der Materialvorrat von einer jeden zweiten Spule 9 zu einem Flechtkopf 11 hin und von diesem zur jeweils einer nächsten Spule 13 oder einem vielfachen Teilungsabstand von derselben hin gespannt. Auf den Spulen 13 ohne Materialvorrat befindet sich nur ein Hilfsfaden, der zumindest bis zum Flechtkopf 11 hinreicht. Das Ende des Materialvorrats wird mit einem Schloss 14 am Ende des Hilfsfadens so weit wie möglich in der Nähe der Spule des Hilfsfadens verbunden.

Der Flechtkopf 11, der in den zuletzt genannten Figuren detailliert dargestellt wird, weist eine kronenförmige Gestalt auf und ist mit Formelementen 15 versehen, die das Einhängen der Fäden bzw. Drähte 4 ermöglichen. Die Formelemente 15 lassen sich versenken, um das Geflecht 2 aushängen und abstreifen zu können. Der Flechtkopf 11 ist im Zentrum der Umlaufbahn von Flügelrädern 16 in einer axialen Anordnung derart platziert, dass die Fäden bzw. Drähte 4 in einem flachen Winkel nach unten zu Klöppeln 17 der Flechtmaschine 7 gerichtet sind.

Nachdem alle für das Geflecht 2 erforderlichen Drähte 4 verbunden und gestrafft sind, beginnt das Flechten in der bekannten Art und Weise, indem die Flügelräder 16 um das Zentrum rotieren, während die Klöppel 17 von Flügelrad zu Flügelrad wechseln und sich dabei gegenseitig auf Ihren Bahnen kreuzen. Der Vorschub für das Geflecht 2 wird über eine Kurvenscheibe 18 in Abhängigkeit der Umläufe der Flügelräder 16 realisiert. Die Geflechtlänge, die mit diesem Verfahren hergestellt werden kann, ist proportional vom Umfang und Steigung des Geflechts 2 sowie von der Länge des Draht- oder Faden-Endes abhängig, welches mit dem Hilfsfaden verbunden ist. Nach dem Flechten werden die freien Enden gebündelt bzw. gefasst, vom Materialvorrat gekappt sowie vom Hilfsfaden entkoppelt. Das so entstandene kugelförmige oder sackartige Hohlgeflecht 2 ist am Anfang geschlossen und am Ende gebündelt. Das Drahtbündel wird so gefasst, dass darin ein Innengewinde hergestellt werden kann, dass mit dem Führungsdraht eines Einführbesteckes in Eingriff bringbar ist.

In dem sich dann anschließenden, werkstoffabhängigen Unformungs- und Wärmebehandelungsverfahren wird das Geflecht 2 in die gewünschte Form des Occlusionsinstruments 1 gebracht. Die Ausgangsform eignet sich zur Herstellung von Occlusionsinstrumenten 1 zur Behandlung eines Patent Foramen Ovale (PFO), eines Ventrikelsektrum-Defekts (VSD), eines Vorhofseptum-Defekts (ASD) sowie eines Peristierenden Duktus Arteriosus (PDA).

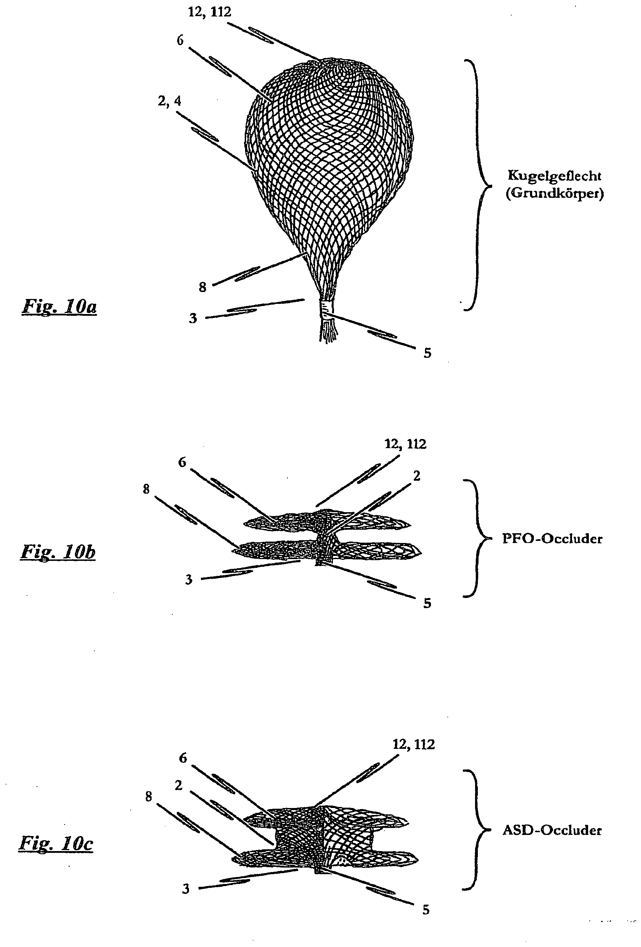

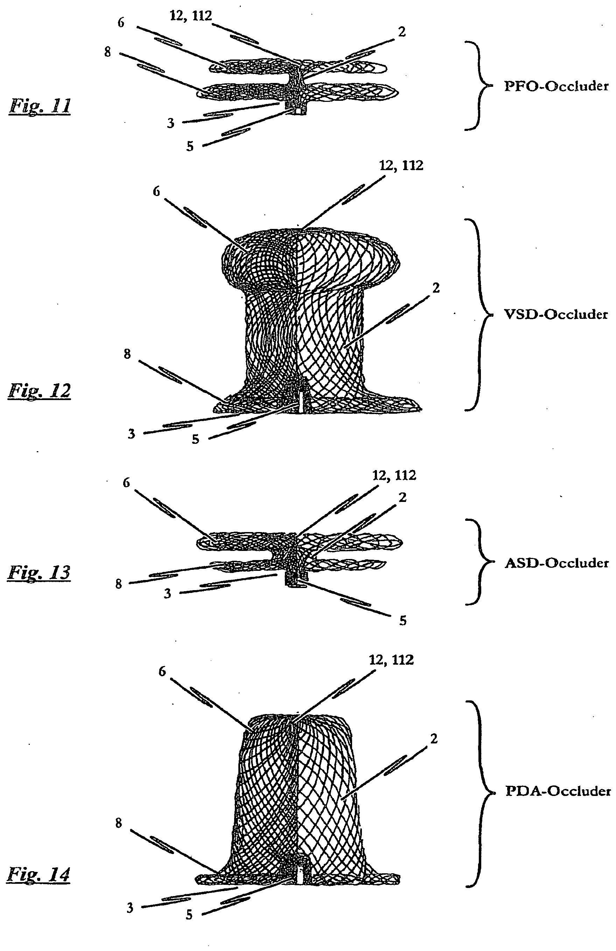

Es sei an dieser Stelle darauf hingewiesen, dass beispielsweise in Fig. 10b eine Seitenansicht eines erfindungsgemäßen Occlusionsinstruments 1 von solch einem PFO-Typ mit einer Seitenschnittdarstellung auf der rechten Seite gezeigt ist, wobei dieses PFO-Occlusionsinstrument 1 aus einem vorstehend beschriebenen Kugelgeflecht 2 hergestellt ist. Des weiteren ist in Fig. 10c eine Seitenansicht eines erfindungsgemäßen Occlusionsinstruments 1 vom ASD-Typ mit einer Seitenschnittdarstellung auf der rechten Seite gezeigt, wobei auch dieses ASD-Occlusionsinstrument 1 aus einem Kugelgeflecht 2 hergestellt ist, deren Herstellung zuvor beschrieben wurde. Ferner ist in Fig. 11 eine weitere Seitenansicht eines erfindungsgemäßen Occlusionsinstruments 1 vom PFO-Typ gezeigt.

Des weiteren sei darauf hingewiesen, dass in Fig. 12 eine Seitenansicht eines erfindungsgemäßen Occlusionsinstruments 1 vom VSD-Typ mit einer Seitenschnittdarstellung auf der rechten Seite offenbart ist, wobei dieses VSD-Occlusionsinstrument 1 aus einem kugelförmigen, birnenförmigen oder tropfenartigen Grundgeflecht 2 hergestellt ist, wie es zuvor beschrieben wurde. Abschließend sei auf die Figuren 13 und 14 verwiesen, in denen jeweils eine Seitenansicht eines erfindungsgemäßen Occlusionsinstruments 1 vom ASD-Typ bzw. PDA-Typ mit jeweils einer Seitenschnittdarstellung auf der rechten Seite dargestellt ist. Diese in Figuren 13 und 14 gezeigten erfindungsgemäßen Occlusionsinstrumente 1 sind wiederum aus einem kugelförmigen, birnenförmigen oder tropfenartigen Grundgeflecht gemäß Fig. 9a bis 9c hergestellt, wobei dieses Grundgeflecht wie vorstehend erläutert hergestellt wird.

Je nach Ausführung werden, von der Fassung 5 aus betrachtet, ein erweiterter Durchmesser (d.h. der distale Retentionsbereich 8) ausgebildet, dem der Steg 10 folgt, an dem sich wieder ein erweiterter, geschlossener Durchmesser (d.h. der proximale Retentionsbereich 6 bzw. die Proximalwand 112) anschließt.

Da unter Umständen das als Grundlage für das Occlusionsinstrument 1 dienende Geflecht 2 als solches einen Defekt nicht vollständig verschließen kann, können Gewebeeinlagen in den Steg 10 und in den sich erweiternden Durchmessern - den distalen und/oder proximalen Retentionsbereichen 6, 8 - eingebracht werden. Jene in bevorzugter Weise aus Dacron bestehende Gewebeeinlagen verschließen dann im eingesetzten Zustand des Occlusionsinstruments 1 die im Geflecht 2 verbleibenden Zwischenräume. Die Gewebeeinlage wird beispielsweise so befestigt, dass sie wie ein Tuch über die proximale Öffnung gespannt werden kann.

Es wird erneut auf Fig. 6 Bezug genommen, wo der Flechtkopf 11 der in Fig. 4 dargestellten Rundflechtmaschine 7 im Detail dargestellt ist, während in Fig. 7 ein Beispiel eines Mittels des in Fig. 6 gezeigten Flechtkopfs 11 hergestellten Geflechts 2 gezeigt ist, welches als Ausgangsgerüst für das erfindungsgemäße Occlusionsinstrument 1 dienen kann. Hier ist deutlich zu erkennen, dass das dem Occlusionsinstrument 1 als Grundkörper dienende Geflecht 2 in Form eines nach oben geschlossenen röhrenförmigen bzw. sackförmigen Geflechts 2 gestaltet wird, welches nur an einem Ende 3 mit einer Fassung 5 versehen werden muss, während von der gegenüberliegenden Seite 12 die Fäden bzw. Drähte 4 beispielsweise aus der Mitte heraus untereinander verflochten werden können.

Das geschlossene Geflecht 2 kann die Form einer Kugel (vgl. Fig. 9a), einer Birne (vgl. Fig. 9b) oder auch eines Tropfens (vgl. Fig. 9c) haben, wobei nur am distalen Ende 3 eine Fassung 5 mit einem Innengewinde zur formflüssigen Verbindung mit einem Einführkatheter vorhanden ist.

Aus den kugelförmigen (Fig. 9a), birnenförmigen (Fig. 9b) und tropfenförmigen (Fig. 9c) Grundgeflechten 2 lassen sich die wichtigsten, aber auch ganz spezifischen Occlusionsinstrumente 1 herstellen, wie sie nachfolgend beschrieben werden, wobei diese erfindungsgemäßen Occlusionsinstrumente 1 mit wesentlich verbesserten funktionalen Eigenschaften versehen sind, wie insbesondere die extrem abgeflachte Form mit der Proximalwand 112 und ohne zusätzliche Narbe im proximalen Retentionsbereich 6 bzw. auf der Proximalwand 112.

Im einzelnen betrifft dies einen Occluder 1 zur Behandlung des Atrium-Septum-Defekts (ASD), was ein Loch in der Vorhofscheidewand des Herzens ist. Ein derartiger Occluder 1 vom ASD-Typ ist beispielsweise in Fig. 13 gezeigt.

Des weiteren lassen sich Occluder 1 zur Behandlung eines Patent-Voramen-Ovale (PFO) herstellen, d.h. zur Behandlung von ovalen Öffnungen/Schlitzen in der Vorhofscheidewand des Herzens. In Fig. 11 ist ein derartiger erfindungsgemäßer PFO-Occluder dargestellt.

Darüber hinaus ist es auch denkbar, erfindungsgemäß einen Occluder zur Behandlung des Peristierenden Duktus Arteriosus (PDA) herzustellen, also zur Behandlung eines offenen Ganges zwischen der Aorta und der Lungenschlagader. In Fig. 14 ist ein derartiger Occluder 1 vom PDA-Typ dargestellt.

Schließlich sei darauf hingewiesen, dass erfindungsgemäß auch Occluder 1 zur Behandlung des Ventrikelsektrum-Defekts (VSD) denkbar wären, d.h. zur Behandlung eines Loches in der Kammerscheidewand des Herzens. Ein Occluder vom VSD-Typ ist beispielsweise in Fig. 12 abgebildet.

Es sei darauf hingewiesen, dass die Ausführung der Erfindung nicht auf die in den Figuren beschriebene Ausführungsbeispiele beschränkt ist, sondern auch in einer Vielzahl von Varianten möglich ist.

### Bezugszeichenliste

- 1: Occlusionsinstrument
- 2: Geflecht
- 3: distales Ende
- 4: Faden, Draht
- 5: Fassung
- 6: proximaler Retentionsbereich
- 7: Flechtmaschine
- 8: distaler Retentionsbereich
- 9: Spule
- 10: Steg
- 11: Flechtkopf
- 12: proximales Ende
- 13: Spule
- 14: Schloss
- 15: Formelement
- 16: Flügelrad
- 17: Klöppel
- 18: Kurvenscheibe

## Patentansprüche

1. Intravaskuläres Occlusionsinstrument (1), insbesondere zur Behandlung von Septum-Defekten, wobei sich das Occlusionsinstrument (1) für eine Zufuhr über einen Katheter zu einem zu verschließenden Defekt eignet und eine stetige, vollständig geschlossene Proximalwand (112) aufweist, wobei kein Material des Occlusionsinstrumentes über die Proximalwand (112) des Occlusionsinstrumentes in den proximalen Bereich des Organs des Patienten hineinragt, und wobei das Occlusionsinstrument aus einem kugelähnlichen oder sackförmigen Hohlgeflecht (2) hergestellt ist, wobei diese sackförmige Hohlgeflecht ein aus Drähten (4) mittels eines Rundflechtverfahrens erzeugtes Hohlgeflecht ist, und wobei das Hohlgeflecht (2) mittels eines Umformungs- und Wärmebehandlungsverfahrens in eine gewünschte Form des Occlusionsinstruments (1) gebracht ist,
**dadurch gekennzeichnet, dass** an dem proximalen Ende (12) des Occlusionsinstruments (1) die Drähte (4) aus der Mitte heraus untereinander verflochten sind und die Enden der Drähte (4) zu seinem distalen Ende (3) zurückgeführt sind, wobei an dem distalen Ende (3) die Enden der Drähte (4) gebündelt sind;
**dass** das Hohlgeflecht einen proximalen Retentionsbereich umfasst, welcher die Proximalwand (112) aufweist, die das proximale Ende bildet, und **dass** das Hohlgeflecht die Proximalwand (112) bildet.

2. Occlusionsinstrument gemäss Anspruch 1, wobei sich das Occlusionsinstrument nahezu unabhängig von der Proportion des Durchmessers des zu verschließenden Defekts an den Defekt anpassbar is, und wobei das Occlusionsinstrument einen proximalen Retentionsbereich mit der Proximalwand aufweist, der im eingesetzten Zustand an dem Randsaum des Defektes vollständig abflacht, und zwar nahezu unabhängig von dem Durchmesser des Defektes und der Stärke der Septumwand.

3. Occlusionsinstrument gemäss Anspruch 1 oder 2, wobei eine Fläche der Proximalwand im Hinblick auf eine Septumwand leicht konvex ausgebildet ist, wodurch eine kraftschlüssige Verbindung zwischen der Proximalwand bzw. einem peripheralen Bereich der Proximalwand mit der Septumwand am zu verschließenden Defekt erreicht wird.

4. Occlusionsinstrument gemäss Anspruch 3, wobei die Proximalwand am peripheralen Bereich leicht konkav geformt ist, während sie in Richtung Mitte, in eine konvexe Formgebung übergeht.

5. Occlusionsinstrument gemäss einem der Ansprüche 1 bis 4, wobei das Hohlgeflecht aus Nitinol oder aus einem anderen Material mit Formgedächtnis- oder Memory-Effekt besteht, und/oder wobei die Proximalwand des Occlusionsinstruments in Gestalt einer Kugelkappe oder eines Tropfensegments gebildet ist.

6. Occlusionsinstrument gemäss einem der Ansprüche 1 bis 4, wobei das Hohlgeflecht aus einer Kupfer-Zink-Aluminium Legierung, Gold-Cadmium-Legierung, oder Legierungen auf einer Eisen-Basis, wie z.B. Eisen-Mangan-Silicium-Legierungen, besteht.

7. Occlusionsinstrument gemäss einem der Ansprüche 1 bis 4, wobei das Hohlgeflecht aus einem Formgedächtnispolymer ausgebildet ist, welches beispielsweise auf Polyanhydriden als Matrix oder auf Polyhydroxycarbonsäuren basierendes biologisch abbaubares Formgedächtnis-Polymermaterial aufweist, insbesondere synthetische, bioabbaubare Implantatmaterialen, und/oder hydrolytisch oder enzymatisch abbaubare Polymere.

8. Occlusionsinstrument gemäss einem der Ansprüche 1 bis 7, das gesamte Occlusionsinstrument aus einem einstückigen Geflecht gebildet ist.

9. Occlusionsinstrument gemäss einem der Ansprüche 1 bis 8, wobei das Occlusionsinstrument einen distalen Retentionsbereich aufweist, welcher ein Verbindungselement aufweist, welches mit einem Katheter in Eingriff bringbar ist, und welches mit einem entsprechend komplementär ausgebildeten Verbindungselement des Katheters kraftschlüssig verbunden werden kann.

10. Occlusionsinstrument gemäss einem der Ansprüche 1-bis 9, wobei vollständig auf Gewebeeinlagen wie beispielsweise Dacron-Einlagen verzichtet ist.

11. Occlusionsinstrument gemäss einem der Ansprüche 1 bis 9, wobei Gewebeeinlagen im Inneren des Occlusionsinstrumentes eingearbeitet sind, welche im Occlusionsinstrument verspannt sind, und wobei das Hohlgeflecht als Grundgerüst für das Occlusionsinstrument dient und eine ausreichende Steifigkeit aufweist, dass es die Gewebeeinlagen aufspannt und diese in ihrer Position verbleiben.

12. Flechtverfahren zur Herstellung eines Occlusionsinstrumentes gemäss einem der Ansprüche 1 bis 11 unter Verwendung eines Flechtkopfes (11), wobei bei dem Verfahren ein Materialvorrat von einer jeden zweiten Spule (13) zu dem Flechtkopf hin und von diesem zur jeweils einer nächsten Spule oder einem vielfachen Teilungsabstand von derselben hin gespannt ist, wobei auf den Spulen ohne Materialvorrat sich nur ein Hilfsfaden befindet, der zumindest bis zum Flechtkopf hinreicht, und das Ende des Materialvorrats mit einem Schloss (14) am Ende des Hilfsfadens so weit wie möglich in der Nähe der Spule des Hilfsfadens verbunden ist, und wobei der Flechtkopf im Zentrum der Umlaufbahn von Flügelrädern in einer axialen Anordnung derart platziert, dass die Fäden bzw. Drähte in einem flachen Winkel nach unten zu Klöppeln (17) der Flechtmaschine gerichtet sind, wobei nachdem alle für ein kugelähnliches oder sackformähnliches Hohlgeflecht erforderlichen Drähte verbunden und gestrafft sind, das Flechten beginnt, indem Flügelräder um das Zentrum rotieren, während die Klöppel von Flügelrad (16) zu Flügelrad wechseln und sich dabei gegenseitig auf Ihren Bahnen kreuzen, und wobei der Vorschub für das Hohlgeflecht über eine Kurvenscheibe (18) in Abhängigkeit der Umläufe der Flügelräder realisiert ist, und wobei die Geflechtlänge, die mit diesem Verfahren hergestellt wird proportional vom Umfang und der Steigung des Hohlgeflechts sowie von der Länge des Draht- oder Faden-Endes bestimmt wird,
wobei der Flechtkopf eine kronenförmige Gestalt aufweist und mit Formelementen versehen ist, die ein Einhängen von Fäden bzw. Drähten ermöglichen, wobei sich die Formelemente versenken lassen, um ein Geflecht auszuhängen und abzustreifen zu können, und das Hohlgeflecht herzustellen , wobei ein gebündeltes distales Ende des Hohlgeflechts dem distalen Ende des fertigen Occlusionsinstruments und dessen gegenüberliegendes geschlossenes Ende dem proximalen Ende des fertigen Occlusionsinstruments entspricht, und eine Flechtkrone umfasst, wobei die Drähte (4) aus der Mitte des Flechtkopfes heraus untereinander verflochten werden, und somit das Hohlgeflecht aus Drähten den (4) mittels eines Rundflechtverfahrens erzeugt wird, wobei an seinem proximalen Ende (12) die Drähte (4) aus der Mitte heraus untereinander verflochten sind und die Ende der Drähte (4) zum distalen Ende (3) zurückgeführt sind, wobei an dem distalen Ende (3) die Enden der Drähte (4) gebündelt werden, wodurch eine stetige, geschlossene Proximalwand ohne hervorragende Anteile geformt wird.

13. Flechtverfahren gemäss Anspruch 12, wobei nach dem Flechten die Enden des Hohlgeflechtes gebündelt oder gefasst werden, vom Materialvorrat gekappt sowie vom Hilfsfaden entkoppelt werden, und anschließend das Hohlgeflecht zur Herstellung des Occlusionsinstrumentes, wie zur Behandlung eines Patent Foramen Ovale (PFO), eines Ventrikelsektrum-Defekts (VSD), eines Vorhofseptum-Defekts (ASD) oder eines Peristierenden Duktus Arteriosus (PDA), in die gewünschte Form des Occlusionsinstruments in einem werkstoffabhängigen Unformungs- und wärmebehandelungsverfahren gebracht werden kann.

## Claims

1. Intravascular occlusion device (1), in particular for the treatment of septal defects, wherein the occlusion device (1) is suitable for delivery via a catheter to a defect that needs to be closed and has a continuous, fully closed proximal wall (112), wherein no material of the occlusion device protrudes beyond the proximal wall (112) of the occlusion device into the proximal area of the patient's organ, and wherein the occlusion device is made from a spherical or bag-shaped hollow mesh (2), wherein this bag-shaped hollow mesh is a hollow mesh made from wires (4) using a circular braiding process, and wherein the hollow mesh (2) is brought into the desired shape of the occlusion device (1) by means of a shaping and heat treatment process,
**characterized in that** at the proximal end (12) of the occlusion device (1), the wires (4) are intertwined from the centre and the ends of the wires (4) are led back to its distal end (3), wherein the ends of the wires (4) are bundled at the distal end (3);
**in that** the hollow mesh comprises a proximal retention area having the proximal wall (112) forming the proximal end, and **in that** the hollow mesh forms the proximal wall (112).

2. Occlusion device according to claim 1, wherein the occlusion device can be adapted to the defect almost independently of the diameter of the defect to be closed, and wherein the occlusion device has a proximal retention area with the proximal wall which, when inserted, flattens out completely at the edge of the defect, almost independently of the diameter of the defect and the thickness of the septal wall.

3. Occlusion device according to claim 1 or 2, wherein a surface of the proximal wall is slightly convex with respect to the septum wall, whereby a non-positive connection is achieved between the proximal wall or a peripheral area of the proximal wall and the septum wall at the defect to be closed.

4. Occlusion device according to claim 3, wherein the proximal wall is slightly concave in the peripheral area, while changing into a convex shape towards the central part.

5. Occlusion device according to one of claims 1 to 4, wherein the hollow mesh is made of nitinol or another material with shape memory, and/or wherein the proximal wall of the occlusion device is formed in the shape of a spherical cap or a teardrop segment.

6. Occlusion device according to one of claims 1 to 4, wherein the hollow mesh consists of a copper-zinc-aluminium alloy, gold-cadmium alloy, or alloys on an iron basis, such as iron-manganese silicon alloys.

7. Occlusion device according to one of claims 1 to 4, wherein the hollow mesh is formed from a shape memory polymer, which has, for example, polyanhydrides as a matrix or biodegradable shape memory polymer material based on polyhydroxy-carboxylic acids, in particular synthetic, biodegradable implant materials, and/or hydrolytically or enzymatically degradable polymers.

8. Occlusion device according to one of claims 1 to 7, wherein the entire occlusion device is formed from a one-piece mesh.

9. Occlusion device according to one of claims 1 to 8, wherein the occlusion device has a distal retention area which has a connecting element which can be engaged with a catheter and which can be non-positively connected to a corresponding complementary connecting element of the catheter.

10. Occlusion device according to one of claims 1 to 9, wherein there are no fabric inserts, such as dacron inserts.

11. Occlusion device according to one of claims 1 to 9, wherein fabric inserts are worked into the interior of the occlusion device, which are braced in the occlusion device, and wherein the hollow mesh serves as a basic framework for the occlusion device and has sufficient rigidity in order to span the fabric inserts and hold them in their position.

12. Braiding method for producing an occlusion device according to one of claims 1 to 11 using a braiding head (11), wherein in this method material is taken from every second spool (13) to the braiding head and from there to the next spool or to the same one using a correspondingly multiplied division distance, wherein there remains only one auxiliary thread on the spools without material, which reaches at least as far as the braiding head, and the end of the material supply is connected to a lock (14) at the end of the auxiliary thread as close as possible to the spool of the auxiliary line, and wherein the braiding head is placed in the centre of the orbit of impellers in an axial arrangement such that such that the wires are directed at a shallow angle downwards towards the spools (17) of the braiding machine, wherein after all the wires required for a spherical or bag-like hollow mesh have been connected and tightened, braiding begins by impellers rotating about the centre while the clapper change from impeller (16) to impeller, crossing each other's paths, and wherein the feed for the hollow mesh is realized via a cam disk (18) depending on the revolutions of the impellers, and wherein the length of the mesh produced by this method is determined proportionally to the circumference and the pitch of the hollow mesh and to the length of the end of the wire or thread,
wherein the braiding head has a crown-shaped form and is provided with shaped elements that allow threads or wires to be hooked in, wherein the shaped elements are sinkable in order to be able to unhook and strip a braid, and to produce the hollow mesh, wherein a bundled distal end of the hollow mesh corresponds to the distal end of the finished occlusion device and its opposite closed end to the proximal end of the finished occlusion device, and comprises a braid crown, whereby the wires (4) are intertwined from the middle of the braiding head, and thus the hollow mesh of wires (4) is produced by means of a circular braiding process, wherein at its proximal end (12) the wires (4) are intertwined from the centre and the ends of the wires (4) are returned to the distal end (3), wherein at the distal end (3) the ends of the wires (4) are bundled, thereby forming a continuous, closed proximal wall without protruding portions.

13. Braiding method according to claim 12, wherein after the braiding the ends of the hollow mesh are bundled or held together, cut from the material and decoupled from the auxiliary thread, and then the hollow mesh is used to produce the occlusion device, such as for treating a patent foramen ovale (PFO), a ventricular septal defect (VSD), an atrial septal defect (ASD) or a persistent ductus arteriosus (PDA), can be brought into the desired shape of the occlusion device in a material-dependent shaping and heat treatment process.

## Revendications

1. Dispositif d'occlusion intravasculaire (1), en particulier destiné au traitement de défauts septaux, où le dispositif d'occlusion (1) est adapté pour être mis en place via un cathéter sur un défaut à fermer et présente une paroi proximale continue, complètement fermée (112), où aucun matériau du dispositif d'occlusion ne dépasse sur la paroi proximale (112) du dispositif d'occlusion (1) dans la zone proximale de l'organe du patient, et où le dispositif d'occlusion est constitué d'un treillis creux sphérique ou en forme de sac (2), où ledit treillis creux en forme de sac est un treillis creux fabriqué à partir de fils (4) au moyen d'un procédé de tressage circulaire, et où le treillis creux (2) est amené au moyen d'un procédé de mise en forme et de traitement thermique dans une forme souhaitée du dispositif d'occlusion (1),
**caractérisé en ce que,** au niveau de l'extrémité proximale (12) du dispositif d'occlusion (1), les fils (4) sont entrelacés à partir du centre et les extrémités des fils (4) sont ramenées vers son extrémité distale (3), où au niveau de l'extrémité distale (3) les extrémités des fils (4) sont regroupées en faisceau ;
**en ce que** le treillis creux comprend une zone de rétention proximale, laquelle présente la paroi proximale (112), qui forme l'extrémité proximale, et **en ce que** le treillis creux forme la paroi proximale (112).

2. Dispositif d'occlusion selon la revendication 1, où le dispositif d'occlusion peut être adapté au défaut quasiment indépendamment de la proportion du diamètre du défaut à fermer, et où le dispositif d'occlusion présente une zone de rétention proximale avec la paroi proximale, qui dans un état d'utilisation au niveau de la couture de bord du défaut est complètement aplatie, à savoir quasiment indépendamment du diamètre du défaut et de la rigidité de la paroi septale.

3. Dispositif d'occlusion selon la revendication 1 ou 2, où une surface de la paroi proximale est légèrement convexe compte tenu d'une paroi septale, grâce à quoi une liaison par friction entre la paroi proximale ou une zone périphérique de la paroi proximale avec la paroi septale est obtenue.

4. Dispositif d'occlusion selon la revendication 3, où la paroi proximale au niveau de la zone périphérique est légèrement concave, alors qu'elle adopte en allant vers le milieu une forme convexe.

5. Dispositif d'occlusion selon l'une des revendications 1 à 4, où le treillis creux est constitué de nitinol ou d'un autre matériau à effet mémoire de forme ou effet mémoire, et/ou où la paroi proximale du dispositif d'occlusion se présente sous la forme d'une calotte sphérique ou d'un segment de goutte.

6. Dispositif d'occlusion selon l'une des revendications 1 à 4, où le treillis creux est constitué d'un alliage cuivre-zinc-aluminium, d'un alliage or-cadmium, ou d'un alliage à base de fer, tel qu'un alliage fer-manganèse-silicium par exemple.

7. Dispositif d'occlusion selon l'une des revendications 1 à 4, où le treillis creux est constitué d'un polymère à mémoire de forme, qui par exemple présente un matériau polymère à mémoire de forme biologiquement dégradable à base de poly(acides hydroxycarboxyliques) ou de polyanhydrides en tant que matrice, en particulier des matériaux d'implant biodégradables synthétiques, et/ou un polymère hydrolytiquement ou enzymatiquement dégradable.

8. Dispositif d'occlusion selon l'une des revendications 1 à 7, où le dispositif d'occlusion entier est formé d'un treillis d'un seul tenant.

9. Dispositif d'occlusion selon l'une des revendications 1 à 8, où le dispositif d'occlusion présente une zone de rétention distale, qui présente un élément de liaison, qui peut aller en prise avec un cathéter, et qui peut se lier par friction avec un élément de liaison de forme complémentaire du cathéter.

10. Dispositif d'occlusion selon l'une des revendications 1 à 9, totalement dénué de doublure textile telle que par exemple une doublure Dacron.

11. Dispositif d'occlusion selon l'une des revendications 1 à 9, où des doublures textiles sont prévues à l'intérieur du dispositif d'occlusion qui sont serrées dans le dispositif d'occlusion, et où le treillis creux sert de squelette pour le dispositif d'occlusion et présente une rigidité suffisante, pour qu'il tende les doublures textiles et que celles-ci restent dans leur position.

12. Procédé de tressage pour la fabrication d'un dispositif d'occlusion selon l'une des revendications 1 à 11 à l'aide d'une tête de tressage (11), où lors du procédé, une alimentation en matériau est tendue de l'une des deux bobines (13) vers la tête de tressage et de celle-ci vers la bobine suivante ou avec un écart de séparation entre les bobines, où sur les bobines sans alimentation en matériau se trouve uniquement un fil auxiliaire, qui s'étend au moins jusqu'à la tête de tressage, et l'extrémité de l'alimentation en matériau est liée avec un fermoir (14) à l'extrémité du fil auxiliaire le plus loin possible au voisinage de la bobine du fil auxiliaire, et où la tête de tressage au centre de l'orbite de la roue est placée selon une disposition axiale de sorte que les fils sont dirigés selon un angle plan vers le bas sous des fuseaux (17) du métier à tresser, où après que tous les fils nécessaires pour un treillis creux sphérique ou en forme de sac ont été reliés et tendus, le tressage commence par rotation des roues autour du centre, tandis que les fuseaux changent de roue (16) en roue et se croisent ainsi sur leurs pistes, et où l'avance pour le treillis creux est réalisée par l'intermédiaire d'une came (18) en fonction des révolutions des roues, et où la longueur du treillis, qui est produite avec ce procédé est proportionnelle à la circonférence et à la pente du treillis creux ainsi qu'à la longueur de l'extrémité de fil, où la tête de tressage présente une forme de couronne et est pourvue d'éléments de forme qui permettent d'attacher les fils, où les éléments de forme peuvent être abaissés pour pouvoir décrocher et enlever un treillis, et fabriquer le treillis creux, où une extrémité distale en faisceau du treillis creux correspond à l'extrémité distale du dispositif d'occlusion terminé et son extrémité fermée opposée correspond l'extrémité proximale du dispositif d'occlusion terminé, et comprend une couronne de tressage, où les fils (4) à partir du centre de la tête de treillis sont entrelacés, et ainsi le treillis creux est réalisé à partir de fils (4) au moyen d'un procédé de tressage circulaire, où au niveau de son extrémité proximale (12) les fils (4) à partir du centre sont entrelacés et les extrémités des fils (4) sont ramenées vers l'extrémité distale (3), grâce à quoi une paroi proximale fermée continue sans excellentes proportions est formée.

13. Procédé de tressage selon la revendication 12, où après le tressage, les extrémités du treillis sont regroupées en faisceau ou maintenues, coupées de l'alimentation en matériau et découplées du fil auxiliaire, puis ensuite le treillis creux destiné à la fabrication du dispositif d'occlusion, par exemple pour traiter un foramen ovale perméable (FOP), une communication interventriculaire (CIV), une communication interauriculaire (CIA) ou un canal artériel persistant (CAP), peut être amené dans la forme souhaitée du dispositif d'occlusion dans un processus de mise en forme et de traitement thermique dépendant du matériau.
